# EUROPÄISCHE PATENTANMELDUNG

(11) **EP 1 760 100 A1**
(43) Veröffentlichungstag der Anmeldung: **07.03.2007**
(21) Anmeldenummer: 05108017.4
(22) Anmeldetag: 01.09.2005
(51) Int. Cl.: C08G 18/80, C08G 18/79, C09J 175/04

(54) **Isocyanatgruppen enthaltende Addukte und Zusammensetzung mit guter Haftung auf Lacksubstraten**

(71) Anmelder: Sika Technology AG, 6340 Baar (CH)
(72) Erfinder: Gimmnich, Peter, 78464, Konstanz (DE); Burckhardt, Urs, 8057, Zürich (CH)
(74) Vertreter: Isler, Jörg

(57) **Zusammenfassung**

Die vorliegende Erfindung betrifft einerseits Addukte der Formel (I), welche erhältlich sind aus der Reaktion eines oligomeren aliphatischen Polyisocyanats und einer Verbindung der Formel HX-R¹. Andererseits betrifft die Erfindung ein- oder zweikomponentige Zusammensetzungen, welche derartige Addukte der Formel (I) enthalten.

Die Addukte der Formel (I) finden als Haftvermittler Verwendung und sind insbesondere geeignet für den Einsatz in elastischen Kleb- und Dichtstoffen.

Die ausgehärteten ein- oder zweikomponentigen Zusammensetzungen weisen eine ausgezeichnete Lackhaftung bei gleichzeitig hoher Dehnbarkeit auf.

## Beschreibung

### Technisches Gebiet

Die vorliegende Erfindung betrifft Isocyanatgruppen enthaltende Addukte sowie derartige Addukte enthaltende Zusammensetzungen.

### Stand der Technik

Es hat sich gezeigt, dass die Haftung von Kleb-, Dichtstoffen und Beschichtungen auf einigen Untergründen sehr schwierig zu erreichen ist.

Lackierte Oberflächen sind ein derartiger Untergrund, auf welchem die Haftung bekanntermassen schwierig zu erreichen ist. Lacke, insbesondere Automobildecklacke, sind als Substrate in dieser Beziehung besonders anspruchsvoll, da sie primär hinsichtlich optischer Erscheinung (Farbe, Glanz) und Beständigkeit gegenüber mechanischer und chemischer Verletzung optimiert sind und deshalb Oberflächeneigenschaften aufweisen, die einen guten Haftungsaufbau in der Regel erschweren.

Es wurden deshalb Bestrebungen gemacht, die Haftung durch den Einsatz eines vorgängig applizierten Voranstrichs, auch als Primer bezeichnet, oder durch den Zusatz von Haftvermittlern zu verbessern. Für eine einfache Applikation werden jedoch von den Anwendern üblicherweise solche Klebstoffe gewünscht, welche eine gute Haftung ohne Primer zum Substrat aufweisen, wie dies beispielsweise im Artikel "Primern nicht notwendig" von M. Rieder, Kleben und Dichten, Vol. 38, Mai 1994, S. 10-17, offenbart wird. Klebstoffe, welche eine gute Haftung auf Lacken aufweisen, wurden verschiedentlich beschrieben, beispielsweise in WO 99/33930. Eine weitere Verbesserung der Haftung ist jedoch notwendig, nicht zuletzt deshalb, weil in der Automobilindustrie immer wieder neue Lacke entwickelt werden, welche erhöhte Anforderungen an die Haftungseigenschaften des Kleb- oder Dichtstoffes stellen.

Der Einsatz von Isocyanuraten als Haftvermittler ist ebenfalls bekannt. US 4,324,879 beschreibt einen verbesserten Prozess zur Trimerisierung von Hexamethylendiisocyanat zum Isocyanurat und erwähnt beispielsweise die überraschend gute Haftung von Lacken enthaltend dieses Polyisocyanat auf Metallen.

Auch Addukte von Polyisocyanaten als Dichtstoffzusatz sind bekannt. US 5,623,044 beschreibt einen Polyurethandichtstoff enthaltend ein Addukt aus einem Polyisocyanat und einem sekundären Amino- oder einem Mercaptosilan, wobei das Addukt im Mittel mindestens eine Silan- und mindestens eine Isocyanatgruppe aufweist, und offenbart, dass der Dichtstoff geeignet ist zum Abdichten von Glas gegenüber Metall.

US 6,649,084 beschreibt einen Isocyanatgruppen enthaltenden Härter für Laminierklebstoffe, welcher unter anderem ein Addukt aus einem aliphatischen Polyisocyanat und einem Block-Polyethylen-Polypropylen-Monol mit einem Molekulargewicht von bevorzugt mindestens 800 enthält.

EP-A-0 540 985 beschreibt hydrophil modifizierte Polyisocyanate, welche als Vernetzer für wässrige Bindemittel geeignet sind und offenbart ein Isocyanatgruppen enthaltendes Addukt aus einem aliphatischen Polyisocyanat und einem monofunktionellen Polyetheralkohol mit im Mittel 5 bis 9.9 Ethylenoxid-Einheiten.

Durch den Einsatz dieser aus dem Stand der Technik bekannten Polyisocyanate oder Addukte wird entweder die Haftung nicht oder nur unwesentlich verbessert, oder sie führen zu Vernetzungsreaktionen und damit einerseits zu einer starken Viskositätserhöhung der nicht ausgehärteten Zusammensetzung und andererseits zu einer starken Reduktion der Elastizität des ausgehärteten Kleb- oder Dichtstoffes.

Für den Einsatz von Polyurethanen als Kleb- oder Dichtstoffe ist es jedoch wesentlich, dass diese Kleb- und Dichtstoffe elastisch sind.

### Darstellung der Erfindung

Aufgabe der vorliegenden Erfindung ist es, Substanzen zur Verfügung zu stellen, welche Klebstoff-, Dichtstoff- oder Beschichtungszusammensetzungen zugefügt werden können und in der Lage sind, deren Haftung auf Lacken zu verbessern, ohne dass nach Aushärtung die Bruchdehnung stark reduziert wird, und sich somit elastische Verklebungen, Abdichtungen und Beschichtungen realisieren lassen, welche auch ohne eine vorgängige Anwendung eines Primers gute Haftung auf Lacken, insbesondere Automobildecklacken, aufweisen.

Überraschenderweise wurde nun gefunden, dass Addukte gemäss Anspruch 1 diese Aufgabe lösen. Insbesondere war überraschend, dass derartige Addukte trotz der Abwesenheit von Silangruppen zu einer derartigen Verbesserung der Haftung auf Lacken führen können.

Es wurde gefunden, dass sich diese Addukte sowohl in einkomponentigen Zusammensetzungen gemäss Anspruch 8 als auch in zweikomponentigen Zusammensetzungen gemäss Anspruch 17 einsetzen lassen, und die gewünschten Eigenschaften erreicht werden. Ausgehärtete derartige Zusammensetzungen weisen neben guter Lackhaftung noch weitere wichtige mechanische Eigenschaften auf, insbesondere eine grosse Zugfestigkeit bei hoher Dehnbarkeit.

Die Zusammensetzungen sind somit geeignet für die elastische Verklebung, Abdichtung und Beschichtung von Lacksubstraten, insbesondere Automobildecklacken, und finden deshalb insbesondere im Fahrzeugbau Anwendung.

### Wege zur Ausführung der Erfindung

Gegenstand der Erfindung sind Addukte der Formel (I).

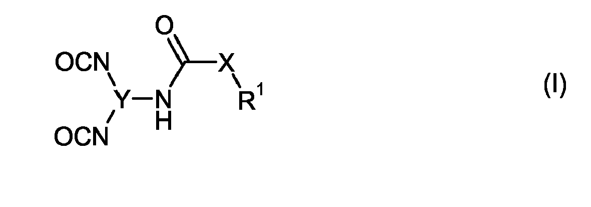

Hierbei steht R¹ für einen Kohlenwasserstoffrest mit 1 bis 20 C-Atomen. R¹ kann gegebenenfalls bis zu 2 Heteroatome enthalten. Der Substituent R¹ enthält weiterhin keine Silangruppe.

Weiterhin steht X in Formel (I) für einen Substituenten

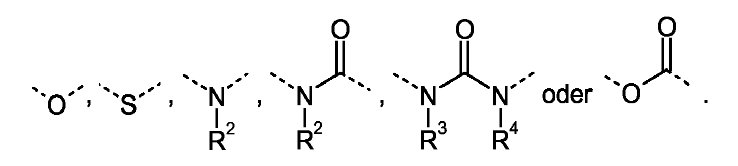

Die gestrichelten Linien stellen hierbei formell die Bindungen zu C=O und R¹ dar.

Weiterhin steht R² für einen linearen oder verzweigten Kohlenwasserstoffrest mit 1 bis 20 C-Atomen. Dieser Kohlenwasserstoffrest weist gegebenenfalls cyclische Anteile auf. Dieser Kohlenwasserstoffrest kann weiterhin gegebenenfalls mindestens eine funktionelle Gruppe aufweisen, welche ausgewählt ist aus der Gruppe umfassend Ether, Sulfon, Nitril, Nitro, Carbonsäureester, Sulfonsäureester und Phosphonsäureester.

Weiterhin stehen R³ und R⁴ entweder unabhängig voneinander für einen linearen oder verzweigten Kohlenwasserstoffrest mit 1 bis 20 C-Atomen, welcher gegebenenfalls cyclische Anteile aufweist.

Oder R³ und R⁴ bilden zusammen mit der Harnstoffgruppe einen fünf- oder sechsgliedrigen Ring. Dieser Ring kann substituiert sein und weist 3 bis 20 C-Atome auf.

Weiterhin steht Y für den Rest eines oligomeren aliphatischen Polyisocyanates mit drei Isocyanatgruppen nach Entfernung aller Isocyanatgruppen.

Mit dem Begriff "Organoalkoxysilan" oder kurz "Silan" werden im vorliegenden Dokument Verbindungen bezeichnet, in denen zum einen mindestens eine, üblicherweise zwei oder drei Alkoxygruppen direkt an das Silicium-Atom gebunden sind (über eine Si-O-Bindung), und die zum anderen mindestens einen direkt an das Silicium-Atom (über eine Si-C-Bindung) gebundenen organischen Rest aufweisen. Entsprechend dazu bezeichnet der Begriff "Silangruppe" die an den organischen Rest des Organoalkoxysilans gebundene Silicium-haltige Gruppe. Die Organoalkoxysilane, beziehungsweise deren Silangruppen, haben die Eigenschaft, bei Kontakt mit Feuchtigkeit zu hydrolysieren. Dabei bilden sich Organosilanole, das heisst Silicium-organische Verbindungen enthaltend eine oder mehrere Silanolgruppen (Si-OH-Gruppen) und, durch nachfolgende Kondensationsreaktionen, Organosiloxane, das heisst Silicium-organische Verbindungen enthaltend eine oder mehrere Siloxangruppen (Si-O-Si-Gruppen).

Mit "Silangruppe" wird im vorliegenden Dokument eine hydrolysierbare, an den organischen Rest eines Organoalkoxysilans gebundene Silicium-haltige Gruppe bezeichnet.

Mit Begriffen wie "Aminosilan", "Hydroxysilan" und "Mercaptosilan" werden Silane bezeichnet, welche die entsprechende funktionelle Gruppe aufweisen, hier also ein Aminoalkylalkoxysilan, Hydroxyalkylalkoxysilan und Mercaptoalkylalkoxysilan.

Mit einem "Oligomer" ist eine Verbindung gemeint, welche durch die Verknüpfung weniger Monomere aufgebaut ist, wobei Dimere und Trimere bereits als Oligomere bezeichnet werden. Unter einem "aliphatischen oligomeren Polyisocyanat" wird ein einzelnes Oligomer oder ein Gemisch von Oligomeren aus aliphatischen Diisocyanaten verstanden, wobei diese Oligomere aus den gleichen oder verschiedenen Diisocyanaten aufgebaut sein können und bei der Oligomerisierung auch kleine Moleküle wie Wasser oder Kohlendioxid eingebaut oder abgespalten werden können.

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst reaktive oligomere Voraddukte, deren funktionelle Gruppen am Aufbau von Makromolekülen beteiligt sind.

Die Vorsilbe "Poly" in Substanzbezeichnungen wie zum Beispiel "Polyisocyanat" oder "Polyol" weist im vorliegenden Dokument darauf hin, dass die jeweilige Substanz formal zwei oder mehr der in ihrer Bezeichnung vorkommenden funktionellen Gruppe - im Beispiel Isocyanat- oder Hydroxylgruppen - pro Molekül enthält.

Das Addukt der Formel (I) ist beispielsweise herstellbar durch die Umsetzung von mindestens einem aliphatischen oligomeren Polyisocyanat der Formel (II) mit mindestens einer Verbindung der Formel (III),

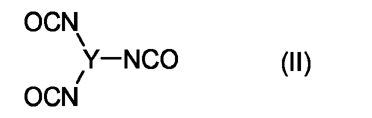

HX-R¹ (III)

Die Substituenten R¹, Y und X weisen die bereits beschriebene Bedeutung auf.

Als aliphatische oligomere Polyisocyanate der Formel (II) geeignet sind Trimere von aliphatischen Diisocyanaten wie beispielsweise die Trimere der folgenden handelsüblichen Diisocyanate:

1,6-Hexamethylendiisocyanat (HDI), 2-Methylpentamethylen-1,5-düsocyanat, 2,2,4- und 2,4,4-Trimethyl-1,6-hexamethylendüsocyanat (TMDI), 1,12-Dodecamethylendiisocyanat, Cyclohexan-1,3- und -1,4-diisocyanat und beliebige Gemische dieser Isomeren, 1-Isocyanato-3,3,5-trimethyl-5-isocyanatomethyl-cyclohexan (=Isophorondüsocyanat oder IPDI), Perhydro-2,4'- und - 4,4'-diphenylmethandiisocyanat (HMDI), 1,4-Diisocyanato-2,2,6-trimethylcyclohexan (TMCDI), m- und p-Xylylendiisocyanat (XDI), 1,3- und 1,4-Tetramethylxylylendüsocyanat (TMXDI), 1,3- und 1,4-Bis-(isocyanatomethyl)-cyclohexan, bevorzugt HDI und IPDI.

Technische Formen dieser Trimeren stellen üblicherweise Gemische von Substanzen mit unterschiedlichen Oligomerisationsgraden und chemischen Strukturen dar. Geeignet sind technische Oligomerengemische, die eine mittlere NCO-Funktionalität von vorzugsweise 2.4 bis 4.0 aufweisen und insbesondere Isocyanurat-, Iminooxadiazindion- oder Biuretgruppen enthalten. Zusätzlich können auch Allophanat-, Carbodiimid-, Uretonimin- oder Oxadiazintrion-Gruppen enthalten sein. Diese Oligomerengemische enthalten bevorzugt mehrheitlich Trimere der Formel (II), insbesondere in Mischung mit Dimeren und den niedrigeren höheren Oligomeren. Geeignete kommerziell erhältliche technische Oligomerengemische von aliphatischen Diisocyanaten sind HDI-Biurete, beispielsweise als Desmodur® N 100 und N 3200 (Bayer), Tolonate^{®} HDB und HDB-LV (Rhodia) und Duranate^{®} 24A-100 (Asahi Kasei); HDI-Isocyanurate, beispielsweise als Desmodur® N 3300, N 3600 und N 3790 BA (alle von Bayer), Tolonate^{®} HDT, HDT-LV und HDT-LV2 (Rhodia), Duranate^{®} TPA-100 und THA-100 (Asahi Kasei) und Coronate^{®} HX (Nippon Polyurethane); HDI-Uretdione, beispielsweise als Desmodur® N 3400 (Bayer); HDI-Iminooxadiazindione, beispielsweise als Desmodur® XP 2410 (Bayer); HDI-Allophanate, beispielsweise als Desmodur® VP LS 2102 (Bayer); sowie IPDI-Isocyanurate, beispielsweise in Lösung als Desmodur^{®} Z 4470 (Bayer) oder in fester Form als Vestanat^{®} T1890/100 (Degussa).

Bevorzugt sind die Trimere von HDI und/oder IPDI, insbesondere die Isocyanurate.

Als Verbindungen der Formel (III) geeignet sind
- Mono-Alkohole, beispielsweise Methanol, Ethanol, Propanol, Isopropanol, n-Butanol, Isobutanol, Pentanol, Hexanol, Isohexanol, Heptanol, Octanol, 2-Ethyl-1-hexanol, Cyclohexanol sowie weitere lineare oder verzweigte oder cyclische Mono-Alkohole mit bis zu 20 C-Atomen, welche bis zu 3 Heteroatome enthalten können, beispielsweise Tetrahydrofurfurylalkohol oder D,L-α,β-Isopropyliden-glycerin (= Solketal^{®});
- Mono-Phenole, beispielsweise Phenol, Kresole und andere Alkyl-Mono-Phenole wie Nonylphenole, 1- und 2-Naphthol und Nitrophenole;
- aliphatische Mono-Thiole, beispielsweise Butanthiol, Hexanthiol, Octanthiol, Cyclohexanthiol oder Benzylmercaptan; Thioglykolester wie Thioglykolsäure-methylester oder Thioglykolsäure-2-ethylhexylester;
- aromatische Mono-Mercaptoverbindungen, beispielsweise 2-Mercaptobenzothiazol oder 2-Mercaptobenzoxazol;
- sekundäre aliphatische Mono-Amine, beispielsweise Dimethylamin, N-Ethylmethylamin, Diethylamin, N-Ethyl-isopropylamin, Dipropylamin, Diisopropylamin, N-Methyl-butylamin, N-Methyl-tert.-butylamin, N-Ethyl-butylamin, Dibutylamin, Diisobutylamin, N-Methyl-hexylamin, N-Methyl-allylamin, N-Methyl-benzylamin, N-tert.Butyl-benzylamin, N-Methyl-cyclohexylamin, N-Allylcyclohexylamin, Dicyclohexylamin, Aziridin, Azetidin, Pyrrolidin, Piperidin, Homopiperidin, Morpholin, Thiomorpholin, 1,2,3,4-Tetrahydroisochinolin, Decahydrochinolin, Perhydroisochinolin sowie weitere sekundäre aliphatische Mono-Amine mit Kohlenwasserstoffketten mit bis zu 20 C-Atomen; sekundäre Mono-Amine basierend auf einem Polyoxypropylen-Gerüst; sowie die Produkte aus der Michael-artigen Addition von primären Mono-Aminen wie beispielsweise Methylamin, Ethylamin, Propylamin, Butylamin, Pentylamin, Hexylamin, Heptylamin, Octylamin, Nonylamin, Decylamin, Allylamin, Cyclohexylamin, Benzylamin sowie weiteren primären Mono-Aminen mit bis zu 20 C-Atomen an Michaelakzeptoren wie Maleinsäurediester, Fumarsäurediester, Citraconsäurediester, Acrylsäureester, Methacrylsäureester, Zimtsäureester, Itaconsäurediester, Vinylphosphonsäurediester, Vinylsulfonsäurearylester, Vinylsulfone, Vinylnitrile, 1-Nitroethylene oder Knoevenagel-Kondensationsprodukte wie beispielsweise solche aus Malonsäurediestern und Aldehyden wie Formaldehyd, Acetaldehyd oder Benzaldehyd, wie beispielsweise N-Butylamino-bernsteinsäure-diethylester;
- sekundäre aromatische Mono-Amine, beispielsweise N-Methyl-anilin, N-Methyl-toluidin und Diphenylamin;
- sekundäre Mono-Carbonsäureamide, beispielsweise N-Methylformamid, N-Methyl-acetamid, sowie Lactame wie Pyrrolidon, Valerolactam und Caprolactam;
- trisubstituierte Mono-Harnstoffe, beispielsweise Trimethylharnstoff, Tributylharnstoff, N-Methyl-N,N'-ethylenharnstoff und N-Methyl-N,N'-propylen-harnstoff;
- Mono-Carbonsäuren, beispielsweise Ameisensäure, Essigsäure, Propionsäure, Buttersäure, Valeriansäure, Capronsäure, 2-Ethylcapronsäure, Laurinsäure oder Benzoesäure.

Bevorzugte Verbindungen der Formel (III) sind aliphatische Mono-Alkohole, insbesondere 2-Ethyl-1-hexanol, aliphatische Mono-Thiole, insbesondere Thioglykolester wie Thioglykolsäure-2-ethylhexylester, und sekundäre aliphatische Mono-Amine, insbesondere Dibutylamin und N-Methylamino-, N-Ethylamino-, N-Propylamino-, N-Butylamino- sowie N-(2-Ethyl-hexyl)-amino-bernsteinsäure-diethylester.

Es ist dem Fachmann klar, dass die Verbindungen der Formel (III) gegenüber aliphatischen Polyisocyanaten mono-funktionell sind, das heisst, dass eine Verbindung der Formel (III) lediglich mit einer Isocyanatgruppe reagiert, oder mit anderen Worten, dass der Substituent R¹ keine NCO-reaktiven Gruppen aufweist.

Nicht geeignet als Verbindungen der Formel (III) sind einerseits Polyethylenglykol-Mono-Alkohole (Polyethylenglykol-Monoether), beziehungsweise Mono-Alkohole basierend auf Copolymeren von Ethylenoxid und Propylenoxid, da aufgrund der bekannten Hydrophilie der Oxyethylen-Einheiten mit derartigen Mono-Alkoholen merkliche Mengen an Wasser eingebracht werden. Dieses Wasser reagiert mit den Isocyanatgruppen und führt zu weiteren Reaktionen, welche zu Viskositätserhöhungen führen, was für den Einsatz in elastischen Klebstoffen, Dichtstoffen oder Beschichtungen nicht erwünscht ist. Die Entfernung des Wassers aus solchen hydrophilen Mono-Alkoholen wäre sehr aufwändig und kostspielig.

Weiterhin ungeeignet als Verbindungen der Formel (III) sind Mono-Alkohole mit insgesamt mehr als 3 Heteroatomen, beispielsweise Mono-Alkohole, die mehr als 2 Ether-Gruppen aufweisen. Auf derartigen Mono-Alkoholen basierende Addukte mit aliphatischen oligomeren Polyisocyanaten führen zu einer deutlich schlechteren Haftung als diejenige mit Addukten nach Formel (I).

Im Falle von Addukten der Formel (I), welche durch die Reaktion zwischen einem aliphatischen oligomeren Polyisocyanat der Formel (II) mit einer Carbonsäure hergestellt wurden, ist dem Fachmann klar, dass die Addukte bei erhöhter Temperatur, beispielsweise bei 60 bis 120 °C, Kohlendioxid abspalten können, wobei Amidgruppen entstehen.

Die Umsetzung erfolgt typischerweise dadurch, dass das aliphatische oligomere Polyisocyanat der Formel (II) und die Verbindung der Formel (III) mit üblichen Verfahren, beispielsweise bei Temperaturen von 0 °C bis 100 °C, gegebenenfalls unter Mitverwendung geeigneter Katalysatoren, zur Reaktion gebracht werden, wobei die Isocyanatgruppen des Polyisocyanates der Formel (II) im Verhältnis zur Isocyanat-reaktiven Gruppe HX der Verbindung der Formel (III) so dosiert sind, dass im Mittel Addukte der Formel (I) mit zwei freien Isocyanatgruppen entstehen. Gegebenenfalls kann das Addukt der Formel (I) unter Mitverwendung von Lösemitteln und/oder Weichmachern hergestellt werden, wobei die verwendeten Lösemittel und Weichmacher keine gegenüber Isocyanaten reaktive Gruppen enthalten dürfen. Insbesondere für den Fall, dass eine der Ausgangssubstanzen der Formeln (II) oder (III) oder das Addukt der Formel (I) bei Raumtemperatur oder bei Reaktionstemperatur eine feste Form aufweisen, ist es vorteilhaft, die Umsetzung in Anwesenheit eines Lösemittels und/oder Weichmachers zu führen, wobei die Verwendung eines Weichmachers bevorzugt ist.

Für die Herstellung des Adduktes der Formel (I) ist es von grossem Vorteil, wenn nahezu wasserfreie oder zumindest stark getrocknete Edukte verwendet werden und der gesamte Herstellprozess sowie die Lagerung des Adduktes der Formel (I) unter weitgehendem Ausschluss von Feuchtigkeit erfolgen.

Typischerweise wird das oligomere aliphatische Polyisocyanat der Formel (II), wie bereits erwähnt, als Bestandteil eines technischen Gemisches mit einer mittleren NCO-Funktionalität von 2.4 bis 4.0 eingesetzt. Bei der Umsetzung eines solchen technischen Gemisches mit einer Verbindung der Formel (III) zu einem Umsetzungsprodukt, welches mindestens ein Addukt der Formel (I) als Bestandteil enthält, wird das stöchiometrische Verhältnis zwischen den NCO-Gruppen und den HX-Gruppen dabei bevorzugt so gewählt, dass das Umsetzungsprodukt eine mittlere NCO-Funktionalität von 1.8 bis 2.6, insbesondere etwa 2, aufweist.

Das Addukt der Formel (I) kann eingesetzt werden als Bestandteil von Zusammensetzungen, insbesondere von Polymeren enthaltenden Zusammensetzungen, wie Primer, Anstriche, Lacke, Klebstoffe, Dichtstoffe, Beschichtungen und Bodenbeläge, insbesondere als Haftvermittler für verschiedene Substrate, beispielsweise anorganische Substrate wie Glas, Glaskeramik, Beton, Mörtel, Backstein, Ziegel, Gips und Natursteine wie Granit oder Marmor; Metalle oder Legierungen wie Aluminium, Stahl, Buntmetalle, verzinkte Metalle; organische Substrate wie Holz, Kunststoffe wie PVC, Polycarbonate, PMMA, Polyester, Epoxidharze; beschichtete Substrate wie pulverbeschichtete Metalle oder Legierungen; sowie Farben und Lacke.

Ein weiterer Gegenstand der vorliegenden Erfindung ist eine einkomponentige Zusammensetzung, welche mindestens ein Addukt der Formel (I), respektive die dafür bevorzugten Ausführungsformen, wie es, respektive sie, oben bereits im Detail beschrieben worden sind, sowie mindestens ein Polymer **P** umfasst.

Das Polymer **P** weist Isocyanatgruppen und gegebenenfalls Silangruppen auf.

In einer Ausführungsform ist das Polymer **P** ein Isocyanatgruppen enthaltendes Polyurethanpolymer **P1.**

In einer anderen Ausführungsform ist das Polymer **P** ein sowohl Isocyanat- als auch Silangruppen enthaltendes Polyurethanpolymer **P2.**

Der Begriff "Polyurethanpolymer" umfasst im vorliegenden Dokument sämtliche Polymere, welche nach dem Diisocyanat-Polyadditions-Verfahren hergestellt werden. Dies schliesst auch solche Polymere ein, die nahezu oder gänzlich frei sind von Urethangruppen, wie Polyether-Polyurethane, Polyester-Polyurethane, Polyether-Polyharnstoffe, Polyharnstoffe, Polyester-Polyharnstoffe, Polyisocyanurate, Polycarbodiimide, usw.

Ein Isocyanatgruppen enthaltendes Polyurethanpolymer P1 ist erhältlich durch die Umsetzung von mindestens einem Polyisocyanat mit mindestens einem Polyol.

Diese Umsetzung kann dadurch erfolgen, dass das Polyol und das Polyisocyanat mit üblichen Verfahren, beispielsweise bei Temperaturen von 50 °C bis 100 °C, gegebenenfalls unter Mitverwendung geeigneter Katalysatoren, zur Reaktion gebracht werden, wobei das Polyisocyanat so dosiert ist, dass dessen Isocyanatgruppen im Verhältnis zu den Hydroxylgruppen des Polyols im stöchiometrischen Überschuss vorhanden sind.

Als Polyole für die Herstellung eines Isocyanatgruppen enthaltenden Polyurethanpolymers können beispielsweise die folgenden handelsüblichen Polyole, oder beliebige Mischungen davon, eingesetzt werden:
- Polyoxyalkylenpolyole, auch Polyetherpolyole oder Oligoetherole genannt, welche Polymerisationsprodukte von Ethylenoxid, 1,2-Propylenoxid, 1,2- oder 2,3-Butylenoxid, Tetrahydrofuran oder Mischungen davon sind, eventuell polymerisiert mit Hilfe eines Startermoleküls mit zwei oder mehreren aktiven Wasserstoffatomen wie beispielsweise Wasser, Ammoniak oder Verbindungen mit mehreren OH- oder NH-Gruppen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.
   Besonders geeignet sind Polyoxyalkylendiole oder Polyoxyalkylentriole, insbesondere Polyoxypropylendiole oder Polyoxypropylentriole.
   Speziell geeignet sind Polyoxyalkylendiole oder Polyoxyalkylentriole mit einem Ungesättigtheitsgrad tiefer als 0.02 mEq/g und mit einem Molekulargewicht im Bereich von 1'000 - 30'000 g/mol, sowie Polyoxypropylendiole und -triole mit einem Molekulargewicht von 400 - 8'000 g/mol. Der Begriff "Molekulargewicht" bezeichnet im vorliegenden Dokument das Molekulargewichtsmittel Mₙ.
   Ebenfalls besonders geeignet sind sogenannte Ethylenoxid-terminierte ("EO-endcapped", ethylene oxide-endcapped) Polyoxypropylenpolyole. Letztere sind spezielle Polyoxypropylenpolyoxyethylenpolyole, die beispielsweise dadurch erhalten werden, dass reine Polyoxypropylenpolyole, insbesondere Polyoxypropylendiole und -triole, nach Abschluss der Polypropoxylierungsreaktion mit Ethylenoxid weiter alkoxyliert werden und dadurch primäre Hydroxylgruppen aufweisen.
- Styrol-Acrylnitril- oder Acrylnitril-Methylmethacrylat-gepfropfte Polyetherpolyole.
- Polyesterpolyole, auch Oligoesterole genannt, hergestellt beispielsweise aus zwei- bis dreiwertigen Alkoholen wie beispielsweise 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1-Trimethylolpropan oder Mischungen der vorgenannten Alkohole mit organischen Dicarbonsäuren oder deren Anhydride oder Ester wie beispielsweise Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure und Hexahydrophthalsäure oder Mischungen der vorgenannten Säuren, sowie Polyesterpolyole aus Lactonen wie beispielsweise ε-Caprolacton.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Polyacrylat- und Polymethacrylatpolyole.
- Polyhydroxyfunktionelle Polykohlenwasserstoffe, auch Oligohydrocarbonole genannt, wie beispielsweise polyhydroxyfunktionelle Ethylen-Propylen-, Ethylen-Butylen- oder Ethylen-Propylen-Dien-Copolymere, wie sie beispielsweise von der Firma Kraton Polymers hergestellt werden, oder polyhydroxyfunktionelle Copolymere aus Dienen wie 1,3-Butandien oder Diengemischen und Vinylmonomeren wie Styrol, Acrylnitril oder Isobutylen, oder polyhydroxyfunktionelle Polybutadienpolyole, wie beispielsweise solche, die durch Copolymerisation von 1,3-Butadien und Allylalkohol hergestellt werden.
- Polyhydroxyfunktionelle Acrylonitril/Polybutadien-Copolymere, wie sie beispielsweise aus Epoxiden oder Aminoalkoholen und carboxylterminierten Acrylonitril/Polybutadien-Copolymeren (kommerziell erhältlich unter dem Namen Hycar^{®} CTBN von Hanse Chemie) hergestellt werden können.

Diese genannten Polyole weisen ein mittleres Molekulargewicht von 250 - 30'000 g/mol, insbesondere von 1'000 - 30'000 g/mol, und eine mittlere OH-Funktionalität im Bereich von 1.6 bis 3 auf.

Zusätzlich zu diesen genannten Polyolen können kleine Mengen von niedrigmolekularen zwei- oder mehrwertigen Alkoholen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, hydriertes Bisphenol A, dimere Fettalkohole, 1,1,1-Trimethylolethan, 1,1,1-Trimethylolpropan, Glycerin, Pentaerythrit, Zuckeralkohole wie Xylit, Sorbit oder Mannit, Zucker wie Saccharose, andere höherwertige Alkohole, niedrigmolekulare Alkoxylierungsprodukte der vorgenannten zwei- und mehrwertigen Alkohole, sowie Mischungen der vorgenannten Alkohole bei der Herstellung des Polyurethanpolymers mitverwendet werden.

Als Polyisocyanate für die Herstellung eines Isocyanatgruppen enthaltenden Polyurethanpolymers können beispielsweise die folgenden handelsüblichen Polyisocyanate verwendet werden:
Aromatische Polyisocyanate wie 2,4- und 2,6-Toluylendiisocyanat und beliebige Gemische dieser Isomeren (TDI), 4,4'-, 2,4'- und 2,2'-Diphenylmethandüsocyanat und beliebige Gemische dieser Isomeren (MDI), Gemische aus MDI und MDI-Homologen ("polymeres MDI" (PMDI)), 1,3- und 1,4-Phenylendiisocyanat, 2,3,5,6-Tetramethyl-1 ,4-diisocyanatobenzol , Naphthalin-1,5-diisocyanat (NDI), 3,3'-Dimethyl-4,4'-düsocyanatodiphenyl (TODI), Tris-(4-isocyanatophenyl)-methan, Tris-(4-isocyanatophenyl)-thio-phosphat; cycloaliphatische Polyisocyanate wie Cyclohexan-1,3- und -1,4-diisocyanat und beliebige Gemische dieser Isomeren, 1-Isocyanato-3,3,5-trimethyl-5-isocyanatomethyl-cyclohexan (=Isophorondüsocyanat oder IPDI), Perhydro-2,4'- und -4,4'-diphenylmethandiisocyanat (HMDI), 1,4-Diisocyanato-2,2,6-trimethylcyclohexan (TMCDI); aliphatische und araliphatische Polyisocyanate wie 1,4-Tetramethylendiisocyanat, 2-Methylpentamethylen-1,5-diisocyanat, 1,6-Hexamethylendiisocyanat (HDI), 2,2,4- und 2,4,4-Trimethyl-1,6-hexamethylendiisocyanat (TMDI), 1,12-Dodecamethylendiisocyanat, Lysin- und Lysinesterdüsocyanat, 1,3- und 1,4-Bis-(isocyanatomethyl)-cyclohexan (BIC), m- und p-Xylylendiisocyanat (m- und p-XDI), 1,3,5-Tris-(isocyanatomethyl)-benzol, m- und p-Tetramethyl-1,3- und -1,4-xylylendiisocyanat (m- und p-TMXDI), Bis-(1-lsocyanato-1-methylethyl)-naphthalin, α,α,α',α',α",α"-Hexamethyl-1,3,5-mesitylentriisocyanat, Dimer- und Trimerfettsäureisocyanate wie 3,6-Bis-(9-isocyanatononyl)-4,5-di-(1-heptenyl)-cyclohexen (Dimeryldiisocyanat); Oligomere und Polymere der vorgenannten Polyisocyanate, sowie beliebige Mischungen der vorgenannten Polyisocyanate und ihrer Oligomere. Bevorzugt sind MDI, TDI, HDI und IPDI.

Ein sowohl Isocyanat- als auch Silangruppen enthaltendes Polyurethanpolymer **P2** ist beispielsweise erhältlich durch die Umsetzung von mindestens einem, wie vorgängig beschriebenen, Isocyanatgruppen enthaltenden Polyurethan polymer **P1** mit einem Organoalkoxysilan, welches mindestens eine Isocyanat-reaktive Gruppe aufweist. Es ist hierbei darauf zu achten, dass das Organoalkoxysilan in Bezug auf die Isocyanatgruppen des Polyurethanpolymers unterstöchiometrisch eingesetzt wird.

Die Umsetzung von mindestens einem Isocyanatgruppen enthaltenden Polyurethanpolymer **P1** mit einem Silan, welches eine NCO-reaktive Gruppe aufweist, kann dadurch erfolgen, dass das Silan mit den Isocyanatgruppen des Polyurethanpolymers zur Reaktion gebracht wird, gegebenenfalls in Anwesenheit eines geeigneten Katalysators, wobei das Silan so dosiert ist, dass dessen isocyanatreaktive Gruppe in einer unterstöchiometrischen Menge bezogen auf die Isocyanatgruppen des Polyurethan polymers vorhanden ist. Diese Umsetzung kann unmittelbar nach der Herstellung des Isocyanatgruppen enthaltenden Polyurethanpolymers erfolgen, sie kann aber auch zu einem späteren Zeitpunkt stattfinden, beispielsweise bei der Vermischung des Isocyanatgruppen enthaltenden Polyurethan polymers mit weiteren Bestandteilen, beispielsweise bei der Herstellung eines Klebstoffes. Ein sowohl Isocyanat- als auch Silangruppen enthaltendes Polyurethanpolymer **P2** weist typischerweise ein Verhältnis zwischen Isocyanat- und Silangruppen im Bereich von 10/1 bis 1/1, bevorzugt 6/1 bis 2/1, auf.

Als Silane mit einer NCO-reaktiven Gruppe zur Umsetzung mit einem Isocyanatgruppen enthaltenden Polyurethanpolymer **P1** geeignet sind
- Mercaptosilane, wie 3-Mercaptopropyl-trimethoxysilan, 3-Mercaptopropyl-dimethoxymethylsilan sowie deren Analoga mit Ethoxy- oder Isopropoxygruppen anstelle der Methoxygruppen am Silicium;
- Hydroxysilane, wie 3-Hydroxypropyl-trimethoxysilan, 3-Hydroxypropyl-dimethoxymethylsilan sowie deren Analoga mit Ethoxy- oder Isopropoxygruppen anstelle der Methoxygruppen am Silicium;
- Aminosilane mit einer sekundären Aminogruppe - im weiteren auch "sekundäre Aminosilane" genannt -, welche abgeleitet sind von handelsüblichen Aminosilanen mit einer primären Aminogruppe. Handelsübliche Aminosilane mit einer primären Aminogruppe sind beispielsweise 3-Aminopropyl-trimethoxysilan, 3-Aminopropyl-dimethoxymethylsilan, 3-Amino-2-methylpropyl-trimethoxysilan, 4-Aminobutyl-trimethoxysilan, 4-Aminobutyldimethoxymethylsilan, 4-Amino-3-methylbutyl-trimethoxysilan, 4-Amino-3,3-dimethylbutyl-trimethoxysilan, 4-Amino-3,3-dimethylbutyl-dimethoxymethylsilan, 2-Aminoethyl-trimethoxysilan, 2-Aminoethyl-dimethoxymethylsilan, Aminomethyl-trimethoxysilan, Aminomethyl-dimethoxymethylsilan, Aminomethylmethoxydimethylsilan, 7-Amino-4-oxaheptyl-dimethoxymethylsilan, sowie deren Analoga mit Ethoxy- oder Isopropoxygruppen anstelle der Methoxygruppen am Silicium. Geeignete Aminosilane mit einer sekundären Aminogruppe sind dementsprechend die Derivate der beispielhaft erwähnten Aminosilane mit einer primären Aminogruppe, welche am Stickstoffatom einen Kohlenwasserstoffrest, beispielsweise eine Methyl-, Ethyl-, Butyl-, Cyclohexyl- oder Phenylgruppe, tragen; mehrfach silanfunktionelle sekundäre Aminosilane wie beispielsweise Bis(trimethoxysilylpropyl)amin; sowie die Produkte aus der Michael-artigen Addition der beispielhaft erwähnten Aminosilane mit einer primären Aminogruppe an Michaelakzeptoren wie Maleinsäurediester, Fumarsäurediester, Citraconsäurediester, Acrylsäureester, Methacrylsäureester, Zimtsäureester, Itaconsäurediester, Vinylphosphonsäurediester, Vinylsulfonsäurearylester, Vinylsulfone, Vinylnitrile, 1-Nitroethylene oder Knoevenagel-Kondensationsprodukte wie beispielsweise solche aus Malonsäurediestern und Aldehyden wie Formaldehyd, Acetaldehyd oder Benzaldehyd.

Besonders geeignete Aminosilane mit einer sekundären Aminogruppe sind N-Methyl-3-aminopropyl-trimethoxysilan, N-Methyl-3-aminopropyl-dimethoxymethylsilan, N-Ethyl-3-amino-2-methylpropyl-trimethoxysilan, N-Ethyl-3-amino-2-methylpropyl-dimethoxymethylsilan, N-Butyl-3-aminopropyl-trimethoxysilan, N-Butyl-3-aminopropyl-dimethoxymethylsilan, N-Butyl-4-amino-3,3-dimethylbutyl-trimethoxysilan, N-Butyl-4-amino-3,3-dimethylbutyl-dimethoxymethylsilan, N-Cyclohexyl-3-aminopropyl-trimethoxysilan, N-Cyclohexyl-3-aminopropyl-dimethoxymethylsilan, N-Phenyl-3-aminopropyl-trimethoxysilan, N-Cyclohexyl-aminomethyl-trimethoxysilan, N-Phenyl-aminomethyl-trimethoxysilan, N-Phenyl-aminomethyl-dimethoxymethylsilan, die Produkte aus der Michael-artigen Addition von 3-Aminopropyl-trimethoxysilan, 3-Aminopropyl-dimethoxymethylsilan, 4-Amino-3,3-dimethylbutyl-trimethoxysilan, 4-Amino-3,3-dimethylbutyl-dimethoxymethylsilan, Aminomethyl-trimethoxysilan oder Aminomethyl-dimethoxymethylsilan an Maleinsäuredimethyl-, -diethyl- oder -dibutylester, Acrylsäuretetrahydrofurfuryl-, -isobornyl-, -hexyl-, -lauryl-, -stearyl-, 2-hydroxyethyl- oder 3-hydroxypropylester, Phosphonsäuredimethyl-, -diethyl- oder -dibutylester, Acrylnitril, 2-Pentennitril, Fumaronitril oder β-Nitrostyrol, sowie die Analoga der genannten Aminosilane mit Ethoxy- anstelle der Methoxygruppen am Silicium.

Typischerweise ist das Addukt der Formel (I) in einer Menge von 0.1 bis 10 Gewichts-%, bevorzugt 0.3 bis 6 Gewichts-%, und insbesondere 0.5 bis 5 Gewichts-%, bezogen auf die einkomponentige Zusammensetzung, vorhanden.

Es ist hierbei wesentlich, dass die Herstellung des Adduktes der Formel (I) getrennt von der Herstellung des Polymers **P** erfolgt. Das aliphatische oligomere Polyisocyanat der Formel (II), welches zur Herstellung des Adduktes der Formel (I) verwendet wird, soll weder mit einem Polyisocyanat, welches zur Herstellung eines Polymers **P** verwendet wird, noch mit dem Polymer **P** in Kontakt treten, bevor die Umsetzung mit der Verbindung der Formel (III) abgeschlossen ist. Auf diese Weise wird sichergestellt, dass die Isocyanat-reaktive Gruppe HX der Verbindung der Formel (III) ausschliesslich mit den Isocyanatgruppen des aliphatischen oligomeren Polyisocyanats der Formel (II) reagiert. Somit kann für die Herstellung der einkomponentigen Zusammensetzung zuerst das Addukt der Formel (I) hergestellt und in dieses das separat hergestellte Polymer **P** eingemischt werden, oder das separat hergestellte Addukt der Formel (I) kann in das Polymer **P** eingemischt werden.

Es ist vorteilhaft, wenn die einkomponentige Zusammensetzung zusätzlich zum Addukt der Formel (I) und zum Polymer **P** mindestens einen Katalysator **KAT-1** enthält. Als Katalysator **KAT-1** geeignet sind Verbindungen, welche zusammen mit Isocyanatgruppen und gegebenenfalls Silangruppen lagerstabil sind, und welche die zur Aushärtung der Zusammensetzung führenden Reaktionen der Isocyanatgruppen und/oder gegebenenfalls der Silangruppen beschleunigen. Als geeignete Katalysatoren **KAT-1** genannt werden sollen Metallverbindungen, beispielsweise Zinnverbindungen, zum Beispiel Dialkylzinndicarboxylate wie Dibutylzinndiacetat, Dibutylzinn-bis-(2-ethylhexanoat), Dibutylzinndilaurat, Dibutylzinndipalmitat, Dibutylzinndistearat, Dibutylzinndioleat, Dibutylzinndilinolat, Dibutylzinndilinolenat, Dibutylzinndiacetylacetonat, Dibutylzinnmaleat, Dibutylzinn-bis-(octylmaleinat), Dibutylzinnphthalat, Dimethylzinndilaurat, Dioctylzinndiacetat oder Dioctylzinndilaurat, Dialkylzinnmercaptide wie Dibutylzinn-bis-(2-ethylhexyl mercaptoacetat) oder Dioctylzinn-bis-(2-ethylhexyl mercaptoacetat), Dibutylzinndichlorid, Monobutylzinntrichlorid, Alkylzinnthioester, Dibutylzinnoxid, Dioctylzinnoxid, Zinn(II)-carboxylate wie Zinn(II)-octoat, Zinn(II)-2-ethylhexanoat, Zinn(II)-laurat, Zinn(II)-oleat oder Zinn(II)-naphtenat, Stannoxane wie Laurylstannoxan, Bismutverbindungen wie Bismut(III)-octoat, Bismut(III)-neodecanoat oder Bismut(III)-oxinate; schwach basische tertiäre Aminverbindungen wie beispielsweise 2,2'-Dimorpholinodiethylether und andere Morpholinether-Derivate; sowie Kombinationen der genannten Verbindungen, insbesondere von metall- und aminogruppenhaltigen Verbindungen.

Zusätzlich zum Addukt der Formel (I), zum Polymer **P** und zum gegebenenfalls vorhandenen Katalysator **KAT-1** kann die einkomponentige Zusammensetzung weitere Bestandteile enthalten, welche jedoch die Lagerstabilität der Zusammensetzung nicht beeinträchtigen sollen, das heisst, dass sie während der Lagerung solche Reaktionen der Isocyanatgruppen und gegebenenfalls der Silangruppen, welche zur Vernetzung der Zusammensetzung führen, nicht in signifikantem Ausmass auslösen sollten. Insbesondere bedeutet dies, dass solche weiteren Bestandteile kein oder höchstens Spuren von Wasser enthalten, beziehungsweise freisetzen, sollten. Als zusätzliche Bestandteile können unter anderem die folgenden wohlbekannten Hilfs- und Zusatzmittel vorhanden sein:
- Weichmacher, beispielsweise Ester organischer Carbonsäuren oder deren Anhydride, Phthalate, wie beispielsweise Dioctylphthalat oder Düsodecylphthalat, Adipate, wie zum Beispiel Dioctyladipat, Sebacate, Polyole wie beispielsweise Polyoxyalkylenpolyole oder Polyesterpolyole, organische Phosphor- und Sulfonsäureester oder Polybutene;
- Lösemittel, beispielsweise Ketone wie Aceton, Methylethylketon, Diisobutylketon, Acetonylaceton, Mesityloxid, sowie cyclische Ketone wie Methylcyclohexanon und Cyclohexanon; Ester wie Ethylacetat, Propylacetat oder Butylacetat, Formiate, Propionate oder Malonate; Ether wie Ketonether, Esterether und Dialkylether wie Diisopropylether, Diethylether, Dibutylether, Diethylenglykoldiethylether sowie Ethylenglykoldiethylether; aliphatische und aromatische Kohlenwasserstoffe wie Toluol, Xylol, Heptan, Octan sowie unterschiedliche Erdölfraktionen wie Naphtha, White Spirit, Petrolether oder Benzin; halogenierte Kohlenwasserstoffe wie Methylenchlorid; sowie N-alkylierte Lactame wie beispielsweise N-Methylpyrrolidon, N-Cyclohexylpyrrolidon oder N-Dodecylpyrrolidon;
- anorganische und organische Füllstoffe, wie zum Beispiel gemahlene oder gefällte Calciumcarbonate, welche gegebenenfalls mit Stearaten beschichtet sind, insbesondere feinteiliges beschichtetes Calciumcarbonat, Russe, Kaoline, Aluminiumoxide, Kieselsäuren, PVC-Pulver oder Hohlkugeln; Fasern, beispielsweise aus Polyethylen; Pigmente;
- weitere in der Polyurethanchemie übliche Katalysatoren;
- Reaktiwerdünner und Vernetzer, beispielsweise Polyisocyanate wie MDI, PMDI, TDI, HDI, 1,12-Dodecamethylendiisocyanat, Cyclohexan-1,3- oder 1,4-diisocyanat, IPDI, Perhydro-2,4`- und -4,4'-diphenylmethandiisocyanat, 1,3-und 1,4-Tetramethylxylylendiisocyanat, Oligomere und Polymere dieser Polyisocyanate, insbesondere Isocyanurate, Carbodiimide, Uretonimine, Biurete, Allophanate und Iminooxadiazindione der genannten Polyisocyanate, Addukte von Polyisocyanaten mit kurzkettigen Polyolen, sowie Adipinsäuredihydrazid und andere Dihydrazide;
- latente Polyamine wie beispielsweise Polyaldimine, Polyketimine, Polyenamine, Polyoxazolidine, an einen Zeolith adsorbierte oder mikroverkapselte Polyamine sowie Amin-Metallkomplexe, bevorzugt Polyaldimine aus aliphatischen primären Polyaminen und aliphatischen, in α-Stellung trisubstituierten Aldehyden wie beispielsweise 2,2-Dimethyl-3-acyloxy-propanal, insbesondere 2,2-Dimethyl-3-lauroyloxy-propanal, sowie Komplexe zwischen Methylendianilin (MDA) und Natriumchlorid (erhältlich als Dispersion in Diethylhexylphthalat oder Diisodecylphthalat unter dem Handelsnamen Caytur^{®} 21 von Crompton Chemical);
- Trocknungsmittel, wie beispielsweise p-Tosylisocyanat und andere reaktive Isocyanate, Orthoameisensäureester, Calciumoxid; Vinyltrimethoxysilan oder andere schnell hydrolysierende Silane wie beispielsweise Organoalkoxysilane, welche in α-Stellung zur Silangruppe eine funktionelle Gruppe aufweisen, oder Molekularsiebe;
- Rheologie-Modifizierer wie beispielsweise Verdickungsmittel, zum Beispiel Harnstoffverbindungen, Polyamidwachse, Bentonite oder pyrogene Kieselsäuren;
- Haftvermittler, insbesondere Silane wie beispielsweise Epoxysilane, Vinylsilane, (Meth)acrylsilane, Isocyanatosilane, Carbamatosilane, S-(Alkylcarbonyl)-mercaptosilane und Aldiminosilane, sowie oligomere Formen dieser Silane;
- Stabilisatoren gegen Wärme, Licht- und UV-Strahlung; flammhemmende Substanzen;
- oberflächenaktive Substanzen wie beispielsweise Netzmittel, Verlaufsmittel, Entlüftungsmittel oder Entschäumer;
- Biozide wie beispielsweise Algizide, Fungizide oder das Pilzwachstum hemmende Substanzen;

sowie weitere üblicherweise in einkomponentigen Polyurethanzusammensetzungen eingesetzte Substanzen.

Eine einkomponentige Zusammensetzung enthaltend mindestens ein Addukt der Formel (I), mindestens ein Polymer **P** sowie gegebenenfalls weitere Bestandteile wird unter Ausschluss von Feuchtigkeit hergestellt und aufbewahrt. Sie ist lagerstabil, d.h. sie kann unter Ausschluss von Feuchtigkeit in einer geeigneten Verpackung oder Anordnung, wie beispielsweise einem Fass, einem Beutel oder einer Kartusche, über einen Zeitraum von mehreren Monaten bis zu einem Jahr und länger aufbewahrt werden, ohne dass sie sich in ihren Anwendungseigenschaften oder in ihren Eigenschaften nach der Aushärtung in einem für ihren Gebrauch relevanten Ausmass verändert.

Bei der Applikation einer solchen Polymerzusammensetzung auf mindestens einen Festkörper oder Artikel kommen die Silan- und/oder Isocyanatgruppen des Polymers **P** in Kontakt mit Feuchtigkeit. Isocyanatgruppen reagieren mit Feuchtigkeit unter Abspaltung von Kohlendioxid zu Aminogruppen, welche schnell mit weiteren Isocyanatgruppen unter Ausbildung von Harnstoffgruppen weiterreagieren. Die Silangruppen haben die Eigenschaft, bei Kontakt mit Feuchtigkeit zu hydrolysieren. Dabei bilden sich Organosilanole (Silicium-organische Verbindungen enthaltend eine oder mehrere Silanolgruppen, Si-OH-Gruppen) und, durch nachfolgende Kondensationsreaktionen, Organosiloxane (Silicium-organische Verbindungen enthaltend eine oder mehrere Siloxangruppen, Si-O-Si-Gruppen). Durch solche Reaktionen härtet die Zusammensetzung schliesslich zu einem elastischen Material aus; dieser Prozess wird auch als Vernetzung bezeichnet. Das für die Aushärtungsreaktion benötigte Wasser kann entweder aus der Luft stammen (Luftfeuchtigkeit), oder aber die Zusammensetzung kann mit einer Wasser enthaltenden Komponente in Kontakt gebracht werden, zum Beispiel durch Bestreichen, beispielsweise mit einem Abglättmittel, oder durch Besprühen, oder es kann der Zusammensetzung bei der Applikation eine Wasser enthaltende Komponente zugesetzt werden, zum Beispiel in Form einer wasserhaltigen Paste, die, beispielsweise über einen Statikmischer, eingemischt wird. Die Aushärtung der Zusammensetzung erfolgt rasch und vollständig, unabhängig davon, ob das dafür benötigte Wasser aus der Luft stammt oder zugesetzt wird. Die in der praktischen Anwendung besonders wichtige Aushärtungsart über die Luftfeuchtigkeit erfolgt unter geeigneten klimatischen Bedingungen, beispielsweise bei 23 °C und 50% relativer Luftfeuchtigkeit, innerhalb von einigen Tagen vollständig.

Die Aushärtung der Polymerzusammensetzung kann gegebenenfalls auch durch Zufuhr von Wärme beschleunigt werden, insbesondere dann, wenn die Zusammensetzung thermisch latente Polyamine, wie Amin-Metallkomplexe oder mikroverkapselte Polyamine, enthält, welche erst nach Überschreiten einer Aktivierungstemperatur, beispielsweise 80 bis 160 °C, mit dem Polymer P reagieren.

Ein weiterer Aspekt der vorliegenden Erfindung stellt eine zweikomponentige Zusammensetzung dar, welche aus zwei Komponenten **K1** und **K2** besteht.

Die Komponente **K1** umfasst mindestens ein Addukt der Formel (I), beziehungsweise eine bevorzugte Ausführungsform davon, wie es, beziehungsweise sie, oben bereits im Detail beschrieben worden sind, sowie mindestens ein Polyisocyanat.

Die Komponente **K2** umfasst mindestens ein Polyol und/oder mindestens ein Polyamin.

Als Polyisocyanat der Komponente **K1** geeignet sind sowohl die für die Herstellung eines Polymers **P** genannten Polyisocyanate als auch die bereits beschriebenen Isocyanatgruppen enthaltenden Polyurethanpolymere **P1.** Bevorzugt ist PMI ("polymeres MDI"), bekannt beispielsweise unter Handelsnamen wie Desmodur^{®} VL, Desmodur^{®} VL 50, Desmodur^{®} VL R 10, Desmodur^{®} VL R 20, Desmodur^{®} VKS 20 F (alle von Bayer), Isonate^{®} M 309, Voranate^{®} M 229, Voranate M^{®} 580 (alle von Dow) oder Lupranat M 10 R (von BASF), bei Raumtemperatur flüssige Formen von MDI (sogenanntes "modifiziertes MDI"), welche Gemische von MDI mit MDI-Derivaten, wie beispielsweise MDI-Carbodümiden oder MDI-Uretoniminen, darstellen, bekannt beispielsweise unter Handelsnamen wie Desmodur^{®} CD, Desmodur^{®} PF, Desmodur^{®} PC (alle von Bayer), sowie Polyurethanpolymere **P1,** welche unter Verwendung von MDI, HDI, TDI oder HDI hergestellt wurden.

Als Polyol der Komponente **K2** geeignet sind dieselben Polyole, welche bereits als geeignet für die Herstellung eines Polymers **P** genannt wurden. Insbesondere geeignet sind hochfunktionelle Polyole, beispielsweise Triole, Tetrole und höherfunktionelle Polyole; aminhaltige bzw. mit Aminen (beispielsweise Ethylendiamin) gestartete Polyetherpolyole; kurzkettige Polyetherpolyole mit Molekulargewichten von 300 bis 2000; hydrophobe Polyole, insbesondere Fettpolyole wie beispielsweise Ricinusöl oder die unter dem Handelsnamen Sovermol^{®} von Cognis bekannten Polyole; ebenso Diol-Kettenverlängerer wie 1,4-Butandiol, 1,6-Hexandiol, Ethylenglykol, Diethylenglykol, Propylenglykol, Dipropylenglykol, 1,4-Bis-(hydroxyethyl)-hydrochinon, 1,4-Cyclohexandiol oder N,N'-Bis-(hydroxyethyl)-piperazin.

Als Polyamine der Komponente **K2** geeignet sind primäre aliphatische Polyamine wie beispielsweise Ethylendiamin, 1,2- und 1,3-Propandiamin, 2-Methyl-1,2-propandiamin, 2,2-Dimethyl-1,3-propandiamin, 1,3- und 1,4-Butandiamin, 1,3- und 1,5-Pentandiamin, 2-Butyl-2-ethyl-1,5-pentandiamin, 1,6-Hexandiamin (HMDA), 2,2,4- und 2,4,4-Trimethylhexamethylendiamin und Mischungen davon (TMD), 1,7-Heptandiamin, 1,8-Octandiamin, 2,4-Dimethyl-1,8-octandiamin, 4-Aminomethyl-1,8-octandiamin, 1,9-Nonandiamin, 2-Methyl-1,9-nonandiamin, 5-Methyl-1,9-nonandiamin, 1,10-Decandiamin, Isodecandiamine, 1,11-Undecandiamin, 1,12-Dodecandiamin, Methyl-bis-(3-aminopropyl)-amin, 1,5-Diamino-2-methylpentan (MPMD), 1,3-Diaminopentan (DAMP), 2,5-Dimethyl-1,6-hexamethylendiamin, cycloaliphatische Polyamine wie 1,3- und 1,4-Diaminocyclohexan, Bis-(4-aminocyclohexyl)-methan (H₁₂MDA), Bis-(4-amino-3-methylcyclohexyl)-methan, Bis-(4-amino-3-ethylcyclohexyl)-methan, Bis-(4-amino-3,5-dimethylcyclohexyl)-methan, Bis-(4-amino-3-ethyl-5-methylcyclohexyl)-methan (M-MECA), 1-Amino-3-aminomethyl-3,5,5-trimethylcyclohexan (= Isophorondiamin oder IPDA), 2- und 4-Methyl-1,3-diaminocyclohexan und Mischungen davon, 1,3- und 1,4-Bis-(aminomethyl)cyclohexan, 1-Cyclohexylamino-3-aminopropan, 2,5(2,6)-Bis-(aminomethyl)-bicydo[2.2.1]heptan (NBDA, hergestellt von Mitsui Chemicals), 3(4),8(9)-Bis-(aminomethyl)-tricyclo-[5.2.1.0^{2,6}]decan, 3,9-Bis-(3-aminopropyl)-2,4,8,10-tetraoxaspiro[5.5]undecan, 1,3-Xylylendiamin (MXDA), 1,4-Xylylendiamin (PXDA), Ethergruppen-haltige aliphatische Polyamine wie Bis-(2-aminoethyl)ether, 4,7-Dioxadecan-1,10-diamin, 4,9-Dioxadodecan-1,12-diamin und höhere Oligomere davon, Polyoxyalkylen-Polyamine mit theoretisch zwei oder drei Aminogruppen, erhältlich beispielsweise unter dem Namen Jeffamine^{®} (hergestellt von Huntsman Chemicals), sowie Polyaminoamide; sekundäre aliphatische Polyamine wie beispielsweise N,N'-Dibutyl-ethylendiamin; N,N'-Di-tert.butyl-ethylendiamin, N,N'-Diethyl-1,6-hexandiamin, 1-(1-Methylethyl-amino)-3-(1-methylethyl-aminomethyl)-3,5,5-trimethylcyclohexan (Jefflink^{®} 754 von Huntsman), N4-Cyclohexyl-2-methyl-N2-(2-methylpropyl)-2,4-pentandiamin, N,N'-Dialkyl-1,3-xylylendiamin, Bis-(4-(N-alkylamino)-cyclohexyl)-methan, N-alkylierte Polyetheramine, Produkte aus der Michael-artigen Addition der beispielhaft erwähnten primären aliphatischen Polyaminen an Michaelakzeptoren wie Maleinsäurediester, Fumarsäurediester, Citraconsäurediester, Acrylsäureester, Methacrylsäureester, Zimtsäureester, Itaconsäurediester, Vinylphosphonsäurediester, Vinylsulfonsäurearylester, Vinylsulfone, Vinylnitrile, 1-Nitroethylene oder Knoevenagel-Kondensationsprodukte wie beispielsweise solche aus Malonsäurediestern und Aldehyden wie Formaldehyd, Acetaldehyd oder Benzaldehyd; aliphatische Polyamine mit primären und sekundären Aminogruppen, wie beispielsweise N-Butyl-1,6-hexandiamin; sowie primäre und/oder sekundäre aromatische Polyamine wie beispielsweise m- und p-Phenylendiamin, 4,4'-Diaminodiphenylmethan (MDA), 3,3'-Dichloro-4,4'-diaminodiphenylmethan (MOCA), Mischungen von 3,5-Dimethylthio-2,4- und - 2,6-toluylendiamin (erhältlich als Ethacure^{®} 300 von Albemarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)-benzolsulfonamid, 5,5'-Methylendianthranilsäure, Dimethyl-(5,5'-Methylendianthranilat), 1,3-Propylen-bis-(4-aminobenzoat), 1,4-Butylen-bis-(4-aminobenzoat), Polytetramethylenoxid-bis-(4-aminobenzoat) (erhältlich als Versalink^{®} von Air Products), 1,2-Bis-(2-aminophenylthio)-ethan, N,N'-Dialkyl-p-phenylendiamin, N,N'-Dialkyl-4,4'-diaminodiphenylmethan, 2-Methylpropyl-(4-chloro-3,5-diaminobenzoat) und tert.Butyl-(4-chloro-3,5-diaminobenzoat.

Es ist auch möglich, Polyamine in Form von Derivaten einzusetzen, in denen alle oder ein Teil der Aminogruppen blockiert sind und erst nach ihrer Aktivierung durch Hydrolyse und/oder Erwärmen mit Isocyanaten reagieren. Beispiele für solche Polyamin-Derivate mit blockierten Aminogruppen sind Aldimine, Ketimine, Enamine, Oxazolidine, Aminale, Ammoniumcarbonate, Amin-Kohlensäure-Salze (Carbamate) oder Amin-Metallkomplexe. Ebenfalls können an einen Zeolith adsorbierte oder mikroverkapselte Polyamine eingesetzt werden.

Typischerweise ist das Addukt der Formel (I) in einer Menge von 0.1 bis 10 Gewichts-%, bevorzugt 0.3 bis 6 Gewichts-%, und insbesondere 0.5 bis 5 Gewichts-%, bezogen auf die zweikomponentige Zusammensetzung, vorhanden.

Es ist hierbei wesentlich, dass die Herstellung des Adduktes der Formel (I) getrennt von der Herstellung der Komponente **K1** erfolgt. Das aliphatische oligomere Polyisocyanat der Formel (II), welches zur Herstellung des Adduktes der Formel (I) verwendet wird, soll nicht mit einem Polyisocyanat, welches Teil der Komponente **K1** ist, in Kontakt treten, bevor die Umsetzung mit der Verbindung der Formel (III) abgeschlossen ist. Auf diese Weise wird sichergestellt, dass die Isocyanat-reaktive Gruppe HX der Verbindung der Formel (III) ausschliesslich mit den Isocyanatgruppen des aliphatischen oligomeren Polyisocyanates der Formel (II) reagiert. Somit kann für die Herstellung der Komponente **K1** zuerst das Addukt der Formel (I) hergestellt und in dieses das Polyisocyanat der Komponente **K1** eingemischt werden, oder das separat hergestellte Addukt der Formel (I) kann in das Polyisocyanat der Komponente **K1** eingemischt werden.

Es ist vorteilhaft, wenn die zweikomponentige Zusammensetzung mindestens einen Katalysator **KAT-2** enthält. Als Katalysator **KAT-2** geeignet sind Verbindungen, welche die Aushärtung der Polymerzusammensetzung beschleunigen. Als geeignete Katalysatoren **KAT-2** genannt werden sollen einerseits die bereits genannten Katalysatoren **KAT-1,** sowie weitere Katalysatoren, beispielsweise Verbindungen von Zink, Mangan, Eisen, Chrom, Cobalt, Kupfer, Nickel, Molybdän, Blei, Cadmium, Quecksilber, Antimon, Vanadium, Titan, Zirconium oder Kalium, wie Zink(II)-acetat, Zink(II)-2-ethylhexanoat, Zink(II)-laurat, Zink(II)-oleat, Zink(II)-naphthenat, Zink(II)-acetylacetonat, Zink(II)-salicylat, Mangan(II)-2-ethylhexanoat, Eisen(III)-2-ethylhexanoat, Eisen(III)-acetylacetonat, Chrom(III)-2-ethylhexanoat, Cobalt(II)-naphthenat, Cobalt(II)-2-ethylhexanoat, Kupfer(II)-2-ethylhexanoat, Nickel(II)-naphthenat, Phenylquecksilber-neodecanoat, Blei(II)-acetat, Blei(II)-2-ethylhexanoat, Blei(II)-neodecanoat, Blei(II)-acetylacetonat, Aluminiumlactat, Aluminiumoleat, Aluminium(III)-acetylacetonat, Diisopropoxytitan-bis-(ethyl-acetoacetat), Dibutoxytitan-bis-(ethylacetoacetat), Dibutoxytitan-bis-(acetylacetonat), Kalium-acetat, Kalium-octoat; tertiäre Aminverbindungen wie Triethylamin, Tributylamin, N-Ethyl-diisopropylamin, N,N,N',N'-Tetramethylethylendiamin, Pentamethyl-diethylentriamin und höhere Homologe davon, N,N,N',N'-Tetramethyl-propylendiamin, Pentamethyl-dipropylentriamin und höhere Homologe davon, N,N,N',N'-Tetramethyl-1,3-butandiamin, N,N,N',N'-Tetramethyl-1,6-hexandiamin, Bis-(dimethylamino)-methan, N,N-Dimethylbenzylamin, N,N-Dimethylcyclohexylamin, N-Methyl-dicyclohexylamin, N,N-Dimethyl-hexadecylamin, Bis-(N,N-diethylaminoethyl)-adipat, N,N-Dimethyl-2-phenylethylamin, Tris-(3-dimethylaminopropyl)-amin, 1,4-Diazabicyclo[2.2.2]octan, 1,8-Diazabicyclo[5.4.0]undec-7-en (DBU), N-Methylmorpholin, N-Ethylmorpholin, N-Cocomorpholin, N,N'-Dimethylpiperazin, N-Methyl-N'-dimethylaminoethyl-piperazin, Bis-(dimethylaminoethyl)-piperazin, 1,3,5-Tris-(dimethylaminopropyl)-hexahydrotriazin oder Bis-(2-dimethylaminoethyl)-ether; stickstoffaromatische Verbindungen wie 4-Dimethylamino-pyridin, N-Methylimidazol, N-Vinylimidazol oder 1,2-Dimethylimidazol; Amidine und Guanidine wie 1,1,3,3-Tetramethylguanidin; tertiäre Aminverbindungen enthaltend aktive Wasserstoffatome, wie Triethanolamin, Triisopropanolamin, N-Methyldiethanolamin, N,N-Dimethylethanolamin, 3-(Dimethylamino)-propyl-diisopropanolamin, Bis-(3-(dimethylamino)-propyl)-isopropanolamin, Bis-(3-dimethylaminopropyl)amin, 3-(Dimethylamino)-propyl-harnstoff, Mannich-Basen wie 2,4,6-Tris-(dimethylaminomethyl)-phenol oder 2,4,6-Tris-(3-(dimethylamino)-propylaminomethyl)-phenol, N-Hydroxypropylimidazol, N-(3-Aminopropyl)-imidazol, sowie Alkoxylierungs- und Polyalkoxylierungsprodukte dieser Verbindungen, beispielsweise Dimethylaminoethoxyethanol; organische Ammoniumverbindungen wie Benzyltrimethylammoniumhydroxid oder alkoxylierte tertiäre Amine; sogenannte "delayed action"-Katalysatoren, welche Modifizierungen bekannter Metall- oder Aminkatalysatoren darstellen, wie Umsetzungsprodukte aus tertiären Aminen und Carbonsäuren oder Phenolen, beispielsweise aus 1,4-Diazabicyclo[2.2.2]octan oder DBU und Ameisensäure oder Essigsäure; sowie Kombinationen der genannten Verbindungen, insbesondere von metall- und aminogruppenhaltigen Verbindungen.

Zusätzlich zum Addukt der Formel (I), zum Polyisocyanat, zum Polyol und/oder Polyamin und zum gegebenenfalls vorhandenen Katalysator **KAT-2** kann die zweikomponentige Zusammensetzung weitere Bestandteile enthalten, wobei dieselben Weichmacher, Lösemittel, Füllstoffe, Katalysatoren, Reaktivverdünner und Vernetzer, latente Polyamine, Trocknungsmittel, Rheologiemodifizierer, Haftvermittler, Stabilisatoren, oberflächenaktiven Substanzen und Biocide eingesetzt werden können, welche bereits für die einkomponentige Zusammensetzung genannt wurden, sowie weitere üblicherweise in zweikomponentigen Polyurethanzusammensetzungen eingesetzte Substanzen. Die Aufteilung dieser zusätzlichen Bestandteile auf die Komponenten **K1** und **K2** erfolgt in der dem Fachmann für zweikomponentige Polyurethanzusammensetzung bekannten Art und Weise.

Die Komponenten **K1** und **K2** sind, getrennt voneinander aufbewahrt, jeweils lagerstabil. Insbesondere die Komponente **K1** muss unter Ausschluss von Feuchtigkeit hergestellt und gelagert werden.

Die beiden Komponenten **K1** und **K2** werden erst kurz vor der Applikation auf eine geeignete Weise miteinander vermischt, wobei darauf geachtet werden muss, dass beim Mischvorgang möglichst wenig Luft in die gemischte Zusammensetzung gelangt, und dass ein geeignetes Mischungsverhältnis eingehalten wird. Sobald die beiden Komponenten miteinander in Kontakt kommen, beginnen die in ihnen enthaltenen reaktiven Bestandteile miteinander zu reagieren und führen so zur Aushärtung der gemischten zweikomponentigen Zusammensetzung. Insbesondere reagieren die Isocyanatgruppen der Komponente **K1** mit den Hydroxyl- und/oder Amingruppen der Komponente **K2.** Die Aushärtung der gemischten zweikomponentigen Zusammensetzung kann bei Raumtemperatur erfolgen; gegebenenfalls kann sie auch durch Zufuhr von Wärme beschleunigt werden, insbesondere dann, wenn die Zusammensetzung langsam reagierende Polyole oder Polyisocyanate enthält, oder wenn sie thermisch latente Polyamine, wie Amin-Metallkomplexe oder mikroverkapselte Polyamine, enthält, welche erst nach Überschreiten einer Aktivierungstemperatur, beispielsweise 80 bis 160 °C, mit dem Polymer **P** reagieren.

Das Mischungsverhältnis zwischen den Komponenten **K1** und **K2** wird üblicherweise so gewählt, dass ein gewisser Überschuss an Isocyanatgruppen in Bezug auf mit Isocyanatgruppen reagierende Gruppen wie Hydroxylgruppen und Aminogruppen vorhanden ist. Üblicherweise wird das Mischungsverhältnis so gewählt, dass das Verhältnis ([OH] + [NH])/[NCO] einen Wert von 0.5 bis 0.95 aufweist. Dadurch wird sichergestellt, dass die gemischte zweikomponentige Zusammensetzung zu einem polymeren Material aushärtet, wobei überschüssige Isocyanatgruppen entweder mit Feuchtigkeit aus der Komponente **K2** oder mit Feuchtigkeit aus der Luft reagieren. Ebenfalls muss darauf geachtet werden, dass zwischen dem Vermischen der Komponenten **K1** und **K2** und dem Applizieren auf eine Oberfläche eines Substrates nicht zuviel Zeit vergeht, da ein zu starkes Vorreagieren vor der Applikation das Ausbilden einer guten Haftung zum Untergrund erschwert.

Die ein- oder zweikomponentigen Zusammensetzungen verfügen im ausgehärteten Zustand über sehr gute mechanische Eigenschaften, insbesondere über eine hohe Dehnbarkeit, welche in einer Vielzahl von Anwendungen, insbesondere in jenen als elastischer Klebstoff, elastischer Dichtstoff oder elastische Beschichtung, von grosser Bedeutung ist.

Die einkomponentigen und zweikomponentigen Zusammensetzungen sind breit einsetzbar beispielsweise als Klebstoffe, Dichtstoffe oder Beschichtungen.

Geeignete Anwendungen sind beispielsweise das Verkleben von Bauteilen im Hoch- oder Tiefbau und bei der Fertigung oder Reparatur von industriellen Gütern oder Konsumgütern, insbesondere von Transportmitteln wie Fahrzeugen zu Wasser oder zu Lande, bevorzugt Automobile, Busse, Lastkraftwagen, Züge oder Schiffe; das Abdichten von Fugen, Nähten oder Hohlräumen in der industriellen Fertigung oder Reparatur, oder im Hoch- oder Tiefbau; sowie das Beschichten von diversen Substraten, beispielsweise als Anstrich, Lack, Primer, Versiegelung oder Schutzbeschichtung, oder als Bodenbelag, beispielsweise für Büros, Wohnbereiche, Spitäler, Schulen, Lagerhallen und Parkgaragen.

In einer bevorzugten Ausführungsform werden die beschriebenen einkomponentigen oder zweikomponentigen Zusammensetzungen als Dichtstoff oder Klebstoff eingesetzt.

In der Anwendung als Klebstoff wird die einkomponentige Zusammensetzung oder die gemischte zweikomponentige Zusammensetzung auf ein Substrat **S1** und/oder ein Substrat **S2** appliziert. Der Klebstoff kann somit auf das eine oder auf das andere Substrat oder auf beide Substrate appliziert werden. Danach werden die zu verklebenden Teile gefügt, worauf der Klebstoff aushärtet. Hierbei ist darauf zu achten, dass das Fügen der Teile innerhalb der so genannten Offenzeit erfolgt, um zu gewährleisten, dass beide Fügeteile verlässlich miteinander verklebt werden.

In der Anwendung als Dichtstoff wird die einkomponentige Zusammensetzung oder die gemischte zweikomponentige Zusammensetzung zwischen die Substrate **S1** und **S2** appliziert und anschliessend erfolgt die Aushärtung. Üblicherweise wird der Dichtstoff in eine Fuge eingepresst.

In beiden Anwendungen kann das Substrat **S1** gleich oder verschieden von Substrat **S2** sein.

Geeignete Substrate **S1** oder **S2** sind beispielsweise anorganische Substrate wie Glas, Glaskeramik, Beton, Mörtel, Backstein, Ziegel, Gips und Natursteine wie Granit oder Marmor; Metalle oder Legierungen wie Aluminium, Stahl, Buntmetalle, verzinkte Metalle; organische Substrate wie Holz, Kunststoffe wie PVC, Polycarbonate, PMMA, Polyester, Epoxidharze; beschichtete Substrate wie pulverbeschichtete Metalle oder Legierungen; sowie Farben und Lacke. Es ist bevorzugt, dass mindestens eines der Substrate **S1** oder **S2** ein Lack, insbesondere ein Automobildecklack, oder eine lackierte Oberfläche ist. Als lackierte Oberflächen gelten insbesondere mit Lack beschichtete Materien wie Holz, mineralische Materialien wie Fels, Beton, Backsteinwerk oder dergleichen, sowie Kunststoffe, Metalle und Metalllegierungen. Insbesondere handelt es sich um ein lackiertes Metallblech.

Unter einem "Lack" wird im vorliegenden Dokument eine ausgehärtete Kunstharz-Beschichtung verstanden, welche auf ein Substrat zum Schutz oder zur Veredelung von dessen Oberfläche aufgebracht ist; der Lack kann dabei transparent sein oder Zuschläge, wie beispielsweise Pigmente, enthalten.

Unter einem "lackierten Blech" wird im vorliegenden Dokument ein dünn ausgewalztes Stück Metall oder Metalllegierung verstanden, welches mit einer oder mehreren Lackschichten beschichtet ist, wobei die oberste Lackschicht auch als "Decklack" bezeichnet wird.

Oft weist ein lackiertes Blech nicht nur eine Lackschicht auf, sondern eine Abfolge von mehreren Lackschichten, die gleich oder verschieden voneinander sein können. Insbesondere im Fahrzeugbau ist es üblich, dass das lackierte Blech mit einem sogenannten Lackaufbau beschichtet ist, bestehend beispielsweise aus einer Grundierung, einer oder mehreren pigmentierten Lackschichten und zuletzt einem transparenten Decklack. Ein typischer Lackaufbau im Fahrzeugbau kann beispielsweise folgendermassen aussehen: 25 µm KTL-Grundierung, 35 µm sogenannter Füller, 18 µm Farblack oder Metallic-Lack und zuletzt 40 µm Klarlack. Beispiele für Lacke, die im Fahrzeugbau Verwendung finden und oft schwierig zu verkleben sind, sind Melamin-Carbamate, silan modifizierte Acryl-Melamine, silanmodifizierte Urethan-Melamine, Hydroxymelamine, Zweikomponenten-Urethane oder säurehärtende Epoxide oder Epoxy-Polyester-Hybride.

Die Substrate können bei Bedarf vor dem Applizieren des Kleb- oder Dichtstoffes vorbehandelt werden. Derartige Vorbehandlungen umfassen insbesondere physikalische und/oder chemische Reinigungsverfahren, beispielsweise Schleifen, Sandstrahlen, Bürsten oder dergleichen, oder Behandeln mit Reinigern oder Lösemitteln oder das Aufbringen eines Haftvermittlers, einer Haftvermittlerlösung oder eines Primers.

Unter einem "Primer" wird im vorliegenden Dokument eine als Voranstrich geeignete Zusammensetzung verstanden, die neben nichtreaktiven flüchtigen Stoffen und optional festen Zuschlägen mindestens ein Polymer und/oder mindestens eine Substanz mit reaktiven Gruppen enthält und die befähigt ist, bei der Applikation auf einem Substrat zu einem festen, gut haftenden Film in einer Schichtdicke von typischerweise 10 - 15 µm auszuhärten, wobei die Aushärtung entweder allein durch das Verdampfen der nichtreaktiven flüchtigen Stoffe, wie beispielsweise Lösemittel oder Wasser, oder durch eine chemische Reaktion, oder durch eine Kombination dieser Faktoren, zustande kommt, und welche eine gute Haftung zu einer nachfolgend aufgebrachten Schicht, beispielsweise einem Klebstoff, aufbaut.

Es wurde festgestellt, dass die einkomponentigen oder zweikomponentigen Zusammensetzungen insbesondere auf Lacken haften, ohne dass vor dem Applizieren des Kleb- oder Dichtstoffes eine Vorbehandlung mittels Haftvermittler oder Primer nötig ist. Es ist somit ein primerloses Kleben und Dichten auf Lacken möglich. Die Applikation des Kleb- oder Dichtstoffes erfolgt vorzugsweise gleichmässig.

Es ist möglich, dass die ein- oder zweikomponentige Zusammensetzung bei erhöhter Temperatur appliziert wird, beispielsweise als Warmschmelzklebstoff (Warm-Melt) bei Temperaturen zwischen 40 und 80°C, oder als Heissschmelzklebstoff (Hot-Melt) bei Temperaturen zwischen 80 und 200°C, insbesondere zwischen 100 und 150°C.

Nach dem Verkleben oder Abdichten der Substrate S1 und S2 mittels einer ein- oder zweikomponentigen Zusammensetzung wird ein verklebter oder abgedichteter Artikel erhalten. Ein derartiger Artikel kann ein Bauwerk, insbesondere ein Bauwerk des Hoch- oder Tiefbaus, oder ein Transportmittel sein. Bevorzugt ist der Artikel ein Transportmittel, beispielsweise ein Fahrzeug zu Wasser oder zu Lande, insbesondere ein Automobil, ein Bus, ein Lastkraftwagen, ein Zug oder ein Schiff, oder ein Anbauteil davon. Besonders bevorzugt ist der verklebte oder abgedichtete Artikel ein Transportmittel, insbesondere ein Automobil, oder ein Anbauteil eines Transportmittels, insbesondere eines Automobils.

In einer bevorzugten Ausführungsform der Erfindung betrifft die Erfindung einen feuchtigkeitshärtenden einkomponentigen Klebstoff, welcher mindestens ein Addukt der Formel (I) sowie mindestens ein Polymer **P**, welches Isocyanatgruppen und gegebenenfalls Silangruppen aufweist, enthält und als Klebstoff für elastische Verklebungen im Fahrzeugbau, insbesondere für das primerlose Verkleben von Lacken, insbesondere von Automobildecklacken aufweisenden Lackblechen, verwendet wird.

Die Elastizität ist für den Einsatz als Klebstoff und Dichtstoff, aber auch als Beschichtung, in den meisten Fällen von wesentlicher Bedeutung. Der Kleb- oder Dichtstoff oder die Beschichtung muss nämlich in der Lage sein, unterschiedliche Abstände von Fügepartner, wie sie beispielsweise durch Masstoleranzen oder Positionierungsunterschiede der Fügepartner oder Fugenpartner verursacht werden, verlässlich zu überbrücken. Auch die Spannungen, eziehungsweise Kräfte, welche durch die unterschiedliche thermische Ausdehnung der Substrate oder durch die statische oder dynamiche Belastung des Klebverbundes, der Abdichtung oder der Beschichtung entstehen, muss der Kleb-, Dichtstoff oder die Beschichtung aufnehmen und übertragen können, ohne dass dabei die innere Kohäsion oder die Haftung mit den Substraten verloren geht. Dichtstoffe verfügen grundätzlich über höhere Dehnbarkeit und tiefere Zugfestigkeit als Klebstoffe. Ein elastischer Klebstoff muss nach der Aushärtung genügend dehnbar sein; in der Regel soll er eine Bruchdehnung von mindestens 300%, insbesondere von mindestens 400%, bevorzugt von mindestens 500%, aufweisen. Mindestwerte für die Zugfestigkeit sind häufig ebenfalls gefordert, typischerweise solche von mindestens 5 MPa, insbesondere mindestens 7 MPa.

Falls die einkomponentige oder zweikomponentige Zusammensetzung als Klebstoff für elastische Verklebungen im Fahrzeugbau verwendet wird, weist sie bevorzugt eine pastöse Konsistenz mit strukturviskosen Eigenschaften auf. Ein solcher Klebstoff wird mittels einer geeigneten Vorrichtung auf das Substrat aufgetragen, bevorzugt in Form einer Raupe, wobei diese eine im Wesentlichen runde oder dreieckige Querschnittsfläche aufweisen kann. Geeignete Methoden zum Auftragen des Klebstoffes sind beispielsweise die Applikation aus handelsüblichen Kartuschen, welche manuell oder mittels Druckluft betrieben werden, oder aus einem Fass oder Hobbock mittels einer Förderpumpe oder eines Extruders, gegebenenfalls mittels eines Applikations-Roboters. Ein Klebstoff mit guten Applikationseigenschaften weist eine hohe Standfestigkeit und einen kurzen Fadenzug auf. Das heisst, er bleibt nach der Applikation in der applizierten Form stehen, fliesst also nicht auseinander, und zieht nach dem Absetzen des Applkationsgerätes keinen oder nur einen sehr kurzen Faden, so dass das Substrat nicht verschmutzt wird.

Elastische Verklebungen im Fahrzeugbau sind beispielsweise das Ankleben von Teilen, wie Kunststoffabdeckungen, Zierleisten, Flansche, Stosstangen, Führerkabinen oder andere Anbauteile, an die lackierte Karosserie eines Transportmittels, oder das Einkleben von Scheiben in die Karosserie. Als Fahrzeuge genannt werden sollen beispielsweise Automobile, Lastkraftwagen, Busse, Schienenfahrzeuge und Schiffe.

Es zeigte sich, dass die erfindungsgemässen Zusammensetzungen enthaltend ein Addukt der Formel (I) ohne Vorbehandlung mittels eines Primers eine deutlich bessere Haftung auf mit Automobildecklacken beschichteten Blechen aufweisen als analoge Zusammensetzungen, welche kein Addukt der Formel (I) enthalten.

### Beispiele

### Beschreibung der Prüfmethoden

**Zugfestigkeit** und **Bruchdehnung** wurden bestimmt an während 7 Tagen im Normklima (23±1 °C, 50±5% relative Luftfeuchtigkeit) ausgehärteten Filmen mit einer Schichtdicke von 2 mm nach DIN EN 53504 (Zuggeschwindigkeit: 200 mm/min).

### Die Haftung wurde wie folgt geprüft:

Ein wie nachfolgend beschrieben lackiertes Blech wurde mit einem mit Isopropanol getränkten Tuch einmal abgewischt.

Nach einer Ablüftezeit von 1 Stunde wurde der jeweilige Klebstoff in Form einer Dreiecksraupe auf das lackierte Blech aufgetragen, wobei die Raupe ungefähr 150 mm in der Länge und ungefähr 10 mm im Durchmesser mass. Das Blech mit der Klebstoffraupe wurde während 7 Tagen im Normklima gelagert, wobei der Klebstoff aushärtete. Darauf wurde die Klebstoffraupe folgendermassen auf Haftung geprüft:
Die Klebstoffraupe wurde an einem Ende bis knapp an die Lackoberfläche eingeschnitten. Das eingeschnittene Ende der Raupe wurde von Hand festgehalten und dann vorsichtig und langsam, schälend in Richtung des anderen Raupenendes, von der Lackoberfläche gezogen. Wenn dabei die Haftung so stark war, dass das Raupenende beim Ziehen abzureissen drohte, wurde mittels eines Cutters ein Schnitt senkrecht zur Raupenziehrichtung bis auf die blanke Lackoberfläche angebracht und die Raupe so ein Stück weit abgelöst. Solche Schnitte wurden, wenn nötig, beim Weiterziehen im Abstand von 2 bis 3 mm wiederholt. Auf diese Weise wurde die gesamte Raupe von der Lackoberfläche gezogen bzw. geschnitten. Die Bewertung der Hafteigenschaften erfolgte anhand dem nach dem Abziehen der Raupe auf der Lackoberfläche zurückbleibenden ausgehärteten Klebstoff (Kohäsionsbruch), und zwar durch Abschätzen des kohäsiven Anteils der Haftfläche, gemäss folgender Skala:
   1 = mehr als 95% Kohäsionsbruch
   2 = 75 - 95% Kohäsionsbruch
   3 = 25 - 75% Kohäsionsbruch
   4 = weniger als 25% Kohäsionsbruch
   5 = 0% Kohäsionsbruch (rein adhäsiver Bruch)
   Testresultate mit Kohäsionsbruchwerten von weniger als 75% gelten als ungenügend.

Die **lackierten Bleche** waren beschichtet mit einem Lackaufbau bestehend aus KTL-Grundierung, Füller, Metallic-Lack und Decklack (Klarlack), wobei die Haftprüfungen auf folgenden Decklacken durchgeführt wurden:
Lackblech 1: Decklack = Clearcoat RK-8013 von DuPont
Lackblech 2: Decklack = Clearcoat RK-8046 von DuPont
Lackblech 3: Decklack = Clearcoat RK-8045 von DuPont

### Herstellung der Addukte

### A-1

100 g (0.52 mol NCO-Gruppen) Hexamethylendiisocyanat-Trimer (Isocyanurat-Typ; Desmodur® N 3300, Bayer), 5.5 g (0.17 mol) Methanol und 0.1 g Dibutylzinndichlorid wurden unter Feuchtigkeitsausschluss auf 40°C aufgeheizt und während 5 Stunden bei 40 °C gerührt. Das Produkt wies einen titrimetrisch bestimmten Gehalt an freien Isocyanatgruppen von 13.2 Gewichts-% auf.

### A-2

100 g (0.52 mol NCO-Gruppen) Hexamethylendiisocyanat-Trimer (Isocyanurat-Typ; Desmodur® N 3300, Bayer), 12.6 g (0.17 mol) Isobutanol und 0.1 g Dibutylzinndichlorid wurden unter Feuchtigkeitsausschluss auf 60 °C aufgeheizt und während 5 Stunden bei 60 °C gerührt. Das Produkt wies einen titrimetrisch bestimmten Gehalt an freien Isocyanatgruppen von 12.6 Gewichts-% auf.

### A-3

100 g (0.52 mol NCO-Gruppen) Hexamethylendiisocyanat-Trimer (Isocyanurat-Typ; Desmodur^{®} N 3300, Bayer), 22.0 g (0.17 mol) 2-Ethyl-1-hexanol und 0.1 g Dibutylzinndichlorid wurden unter Feuchtigkeitsausschluss auf 60 °C aufgeheizt und während 2 Stunden bei 60 °C gerührt. Das Produkt wies einen titrimetrisch bestimmten Gehalt an freien Isocyanatgruppen von 11.0 Gewichts-% auf.

### A-4

100 g (0.52 mol NCO-Gruppen) Hexamethylendiisocyanat-Trimer (Isocyanurat-Typ; Desmodur^{®} N 3300, Bayer), 32.4 g (0.17 mol) 2-Butyl-1-octanol, 0.1 g Dibutylzinndichlorid und 60 g Tetraethylenglykoldimethylether wurden unter Feuchtigkeitsausschluss auf 60 °C aufgeheizt und während 2 Stunden bei 60 °C gerührt. Das Produkt wies einen titrimetrisch bestimmten Gehalt an freien Isocyanatgruppen von 7.2 Gewichts-% auf.

### A-5

100 g (0.52 mol NCO-Gruppen) Hexamethylendiisocyanat-Trimer (Isocyanurat-Typ; Desmodur^{®} N 3300, Bayer), 23.1 g (0.17 mol) D,L-α,β-Isopropyliden-glycerin (=Solketal^{®}, Fluka), 0.1 g Dibutylzinndichlorid und 51 g Tetraethylenglykoldimethylether wurden unter Feuchtigkeitsausschluss auf 60 °C aufgeheizt und während 2 Stunden bei 60 °C gerührt. Das Produkt wies einen titrimetrisch bestimmten Gehalt an freien Isocyanatgruppen von 7.5 Gewichts-% auf.

### A-6

100 g (0.52 mol NCO-Gruppen) Hexamethylendüsocyanat-Trimer (Isocyanurat-Typ; Desmodur^{®} N 3300, Bayer) und 22.0 g (0.17 mol) Dibutylamin wurden unter Feuchtigkeitsausschluss gemischt, wobei sich die Mischung durch die exotherme Reaktion auf 75 °C aufwärmte. Es wurde während einer Stunde gerührt. Das Produkt wies einen titrimetrisch bestimmten Gehalt an freien Isocyanatgruppen von 10.7 Gewichts-% auf.

### A-7

100 g (0.52 mol NCO-Gruppen) Hexamethylendiisocyanat-Trimer (Isocyanurat-Typ; Desmodur^{®} N 3300, Bayer), 34.8 g (0.17 mol) Thioglykolsäure-2-ethylhexylester und 0.1 g Dibutylzinndilaurat wurden unter Feuchtigkeitsausschluss auf 60 °C aufgeheizt und während 10 Stunden bei 60 °C gerührt. Das Produkt wies einen titrimetrisch bestimmten Gehalt an freien Isocyanatgruppen von 10.7 Gewichts-% auf.

### A-8

100 g (0.55 mol NCO-Gruppen) Hexamethylendiisocyanat-Trimer (Isocyanurat-Typ; Desmodur^{®} N 3600, Bayer), 21.0 g (0.16 mol) 2-Ethyl-1-hexanol und 0.1 g Dibutylzinndichlorid wurden unter Feuchtigkeitsausschluss auf 60 °C aufgeheizt und während 10 Stunden bei 60 °C gerührt. Das Produkt wies einen titrimetrisch bestimmten Gehalt an freien Isocyanatgruppen von 11.9 Gewichts-% auf.

### A-9

100 g (0.57 mol NCO-Gruppen) Hexamethylendiisocyanat-Trimer (Iminooxadiazindion-Typ; Desmodur^{®} XP 2410, Bayer), 21.0 g (0.16 mol) 2-Ethyl-1-hexanol und 0.1 g Dibutylzinndichlorid wurden unter Feuchtigkeitsausschluss auf 60 °C aufgeheizt und während 10 Stunden bei 60 °C gerührt. Das Produkt wies einen titrimetrisch bestimmten Gehalt an freien Isocyanatgruppen von 13.4 Gewichts-% auf.

### A-10

100 g (0.55 mol NCO-Gruppen) Hexamethylendiisocyanat-Trimer (Biuret-Typ; Desmodur^{®} N 3200, Bayer), 22.7 g (0.18 mol) 2-Ethyl-1-hexanol und 0.1 g Dibutylzinndichlorid wurden unter Feuchtigkeitsausschluss auf 60 °C aufgeheizt und während 10 Stunden bei 60 °C gerührt. Das Produkt wies einen titrimetrisch bestimmten Gehalt an freien Isocyanatgruppen von 11.7 Gewichts-% auf.

### A-11

100 g (0.52 mol NCO-Gruppen) Hexamethylendiisocyanat-Oligomer (Uretdion-Typ; Desmodur^{®} N 3400, Bayer), 13.5 g (0.10 mol) 2-Ethyl-1-hexanol und 0.1 g Dibutylzinndichlorid wurden unter Feuchtigkeitsausschluss auf 60 °C aufgeheizt und während 2 Stunden bei 60 °C gerührt. Das Produkt wies einen titrimetrisch bestimmten Gehalt an freien Isocyanatgruppen von 15.0 Gewichts-% auf.

### A-12

100 g (0.40 mol NCO-Gruppen) Isophorondiisocyanat-Trimer (Isocyanurat-Typ; Vestanat^{®} T1890/100, Degussa), 18.2 g (0.14 mol) 2-Ethyl-1-hexanol, 0.1 g Dibutylzinndichlorid und 60 g Tetraethylenglykoldimethylether wurden unter Feuchtigkeitsausschluss auf 60 °C aufgeheizt und während 8 Stunden bei 60 °C gerührt. Das Produkt wies einen titrimetrisch bestimmten Gehalt an freien Isocyanatgruppen von 5.9 Gewichts-% auf.

### V-1 (Vergleich)

100 g (0.52 mol NCO-Gruppen) Hexamethylendiisocyanat-Trimer (Isocyanurat-Typ; Desmodur^{®} N 3300, Bayer), 36.7 g (0.18 mol) 3-Mercaptopropyl-trimethoxysilan (Silquest^{®} A-189, GE Advanced Materials), 0.1 g Dibutylzinndichlorid und 60 g Tetraethylenglykoldimethylether wurden unter Feuchtigkeitsausschluss auf 60 °C aufgeheizt und während 8 Stunden bei 60 °C gerührt. Das Produkt wies einen titrimetrisch bestimmten Gehalt an freien Isocyanatgruppen von 6.9 Gewichts-% auf.

### V-2 (Vergleich)

100 g (0.52 mol NCO-Gruppen) Hexamethylendiisocyanat-Trimer (Isocyanurat-Typ; Desmodur^{®} N 3300, Bayer), 57.8 g (0.17 mol) Polypropylenglykol-monobutylether mit Molekulargewicht 340, 0.1 g Dibutylzinndichlorid und 70 g Tetraethylenglykoldimethylether wurden unter Feuchtigkeitsausschluss auf 60 °C aufgeheizt und während 8 Stunden bei 60 °C gerührt. Das Produkt wies einen titrimetrisch bestimmten Gehalt an freien Isocyanatgruppen von 6.1 Gewichts-% auf.

### V-3 (Vergleich)

100 g (0.52 mol NCO-Gruppen) Hexamethylendiisocyanat-Oligomer (Uretdion-Typ; Desmodur^{®} N 3400, Bayer), 260 g Polyethylenglykolmonomethylether mit Molekulargewicht 1000 (0.26 mol), 160 g Tetraethylenglykoldimethylether und 0.1 g Dibutylzinndichlorid wurden unter Feuchtigkeitsausschluss auf 60 °C aufgeheizt und während 2 Stunden bei 60 °C gerührt. Das Produkt wies einen titrimetrisch bestimmten Gehalt an freien Isocyanatgruppen von 1.6 Gewichts-% auf. Beim Aufbewahren zeigte sich ein deutlicher Anstieg der Viskosität.

### Herstellung der Klebstoffe

### Beispiel 1 bis 17

In einem Vakuummischer wurden jeweils 100 Gewichtsteile Klebstoff-Grundformulierung mit den in der Tabelle 1 angegebenen Zusätzen in der angegebenen Menge versetzt und zu einer homogenen Paste verarbeitet, welche unter Ausschluss von Feuchtigkeit aufbewahrt wurde. Mit jeder Mischung wurden die in Tabelle 1 angegebenen Prüfungen durchgeführt.

### Die Klebstoff-Grundformulierung wurde wie folgt hergestellt:

In einem Vakuummischer wurden 2000 g Polymer 1, 2100 g Polymer 2, 1100 g Diisodecylphthalat (Palatinol^{®} Z, BASF), 600 g Harnstoff-Verdickungsmittel, 10 g p-Toluol-sulfonylisocyanat (Zusatzmittel TI, Bayer), 2000 g getrockneter Russ, 1700 g calciniertes Kaolin und 4 g Dibutylzinndichlorid zu einer homogenen Paste verarbeitet, welche unter Ausschluss von Feuchtigkeit aufbewahrt wurde.

Das Polymer 1 wurde wie folgt hergestellt:
1295 g Polyol Acclaim^{®} 4200 N (low monol Polyoxypropylen-Diol, OH-Zahl 28.5 mg KOH/g; Bayer), 2585 g Polyol Caradol^{®} MD34-02 (Polyoxypropylenpolyoxyethylen-Triol, OH-Zahl 35.0 mg KOH/g; Shell), 620 g 4,4'-Methylendiphenyldiisocyanat (MDI; Desmodur^{®} 44 MC L, Bayer) und 500 g Diisodecylphthalat (DIDP; Palatinol^{®} Z, BASF) wurden nach bekanntem Verfahren bei 80 °C zu einem NCO-terminierten Polyurethanpolymeren umgesetzt. Das Reaktionsprodukt hatte einen titrimetrisch bestimmten Gehalt an freien Isocyanatgruppen von 2.03 Gewichts-%.

Das Polymer 2 wurde wie folgt hergestellt:
1770 g Polyol Acclaim^{®} 4200 N (low monol Polyoxypropylen-Diol, OH-Zahl 28.5 mg KOH/g; Bayer) und 230 g 4,4'-Methylendiphenyldiisocyanat (MDI; Desmodur^{®} 44 MC L, Bayer) wurden nach bekanntem Verfahren bei 80 °C zu einem NCO-terminierten Polyurethanprepolymeren umgesetzt. Das Reaktionsprodukt hatte einen titrimetrisch bestimmten Gehalt an freien Isocyanatgruppen von 1.97 Gewichts-%.

Das Harnstoff-Verdickungsmittel wurde wie folgt hergestellt:
In einem Vakuummischer wurden 3000 g Düsodecylphthalat (DIDP; Palatinol^{®} Z, BASF) und 480 g 4,4'-Methylendiphenyldiisocyanat (MDI; Desmodur^{®} 44 MC L, Bayer) vorgelegt und leicht aufgewärmt. Dann wurden unter starkem Rühren 270 g Monobutylamin langsam zugetropft. Die entstehende Paste wurde unter Vakuum und Kühlung eine Stunde weitergerührt.

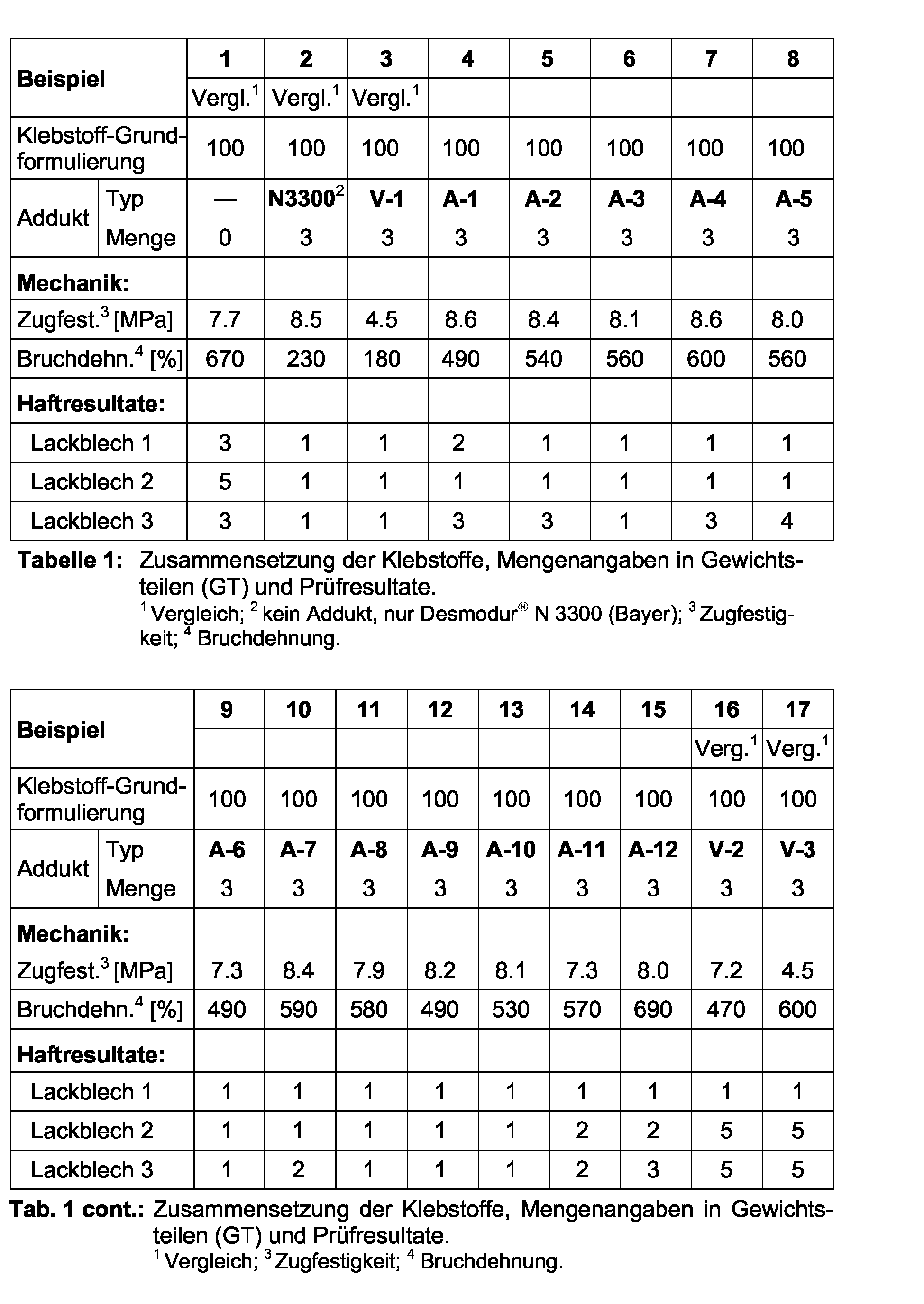

Die Resultate in Tabelle 1 zeigen unter anderem folgendes:

Das Beispiel 1 (Vergleich) ohne Zusatz eines Adduktes der Formel (I) weist wohl einen hohen Bruchdehnungswert, aber eine ungenügende Haftung auf den geprüften Lackblechen auf.

Das Beispiel 2 (Vergleich), welches anstelle eines Adduktes der Formel (I) Desmodur^{®} N 3300 enthält, und das Beispiel 3 (Vergleich), welches das Silangruppen enthaltende Addukt **V-1** enthält, weisen wohl eine gute Haftung auf den geprüften Lackblechen, aber sehr tiefe Bruchdehnungswerte auf, so dass sie als elastische Klebstoffe ungeeignet sind.

Die erfindungsgemässen Beispiele 4 bis 15, welche die Addukte **A-1** bis **A-12** enthalten, weisen alle hohe bis sehr hohe Bruchdehnungswerte und generell gute bis sehr gute Haftung auf den geprüften Lackblechen auf.

Die Beispiele 16 und 17 (Vergleichsbeispiele), welche Addukte aus Mono-Alkoholen mit insgesamt mehr als 3 Heteroatomen enthalten, weisen eine ungenügende Haftung auf den geprüften Lackblechen auf.

## Patentansprüche

1. Addukt der Formel (I) 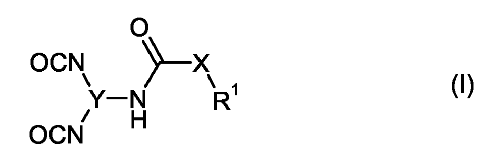 wobei
R¹ für einen Kohlenwasserstoffrest mit 1 bis 20 C-Atomen steht, welcher gegebenenfalls bis zu 2 Heteroatome enthalten kann, und welcher keine Silangruppe enthält; 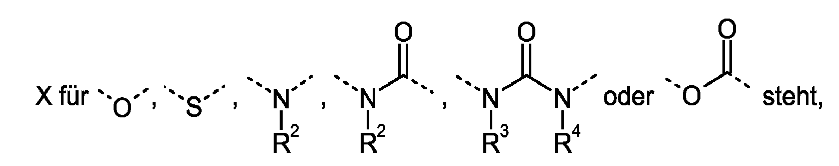 wobei
R² für einen linearen oder verzweigten Kohlenwasserstoffrest mit 1 bis 20 C-Atomen steht, welcher gegebenenfalls cyclische Anteile aufweist, und welcher gegebenenfalls mindestens eine funktionelle Gruppe aufweist, welche ausgewählt ist aus der Gruppe umfassend Ether, Sulfon, Nitril, Nitro, Carbonsäureester, Sulfonsäureester und Phosphonsäureester;
R³ und R⁴ unabhängig voneinander für einen linearen oder verzweigten Kohlenwasserstoffrest mit 1 bis 20 C-Atomen, welcher gegebenenfalls cyclische Anteile aufweist, stehen,
oder R³ und R⁴ zusammen mit der Harnstoffgruppe einen fünf- oder sechsgliedrigen Ring bilden, welcher gegebenenfalls substituiert ist, und welcher 3 bis 20 C-Atome aufweist;
und die gestrichelten Linien die Bindungen zu C=O und R¹ darstellen;
und
Y für den Rest eines oligomeren aliphatischen Polyisocyanates mit drei Isocyanatgruppen nach Entfernung aller Isocyanatgruppen steht.

2. Addukt der Formel (I) gemäss Anspruch 1, **dadurch gekennzeichnet, dass** das Addukt hergestellt wird aus der Reaktion zwischen mindestens einem oligomeren aliphatischen Polyisocyanat der Formel (II) und mindestens einer Verbindung der Formel (III) 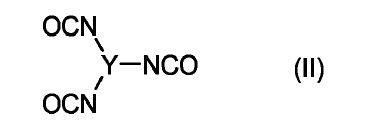
HX-R¹ (III)

3. Addukt der Formel (I) gemäss Anspruch 1 oder 2, **dadurch gekennzeichnet, dass** das oligomere aliphatische Polyisocyanat ein Trimer von 1,6 Hexamethylendiisocyanat (HDI) und/oder Isophorondiisocyanat (IPDI) ist.

4. Addukt der Formel (I) gemäss Anspruch 2 oder 3, **dadurch gekennzeichnet, dass** die Verbindung der Formel (III) ein aliphatischer Mono-Alkohol oder ein aliphatisches Mono-Thiol oder ein sekundäres aliphatisches Mono-Amin ist.

5. Addukt der Formel (I) gemäss Anspruch 4, wobei die Verbindung der Formel (III) 2-Ethyl-1-hexanol oder Thioglykolsäure-2-ethylhexylester oder Dibutylamin oder N-Methylamino-, N-Ethylamino-, N-Propylamino-, N-Butylamino- oder N-(2-Ethyl-hexyl)-amino-bernsteinsäure-diethylester ist.

6. Verfahren zur Herstellung eines Adduktes der Formel (I) gemäss einem der Ansprüche 1 bis 5, bei welchem mindestens ein oligomeres aliphatischen Polyisocyanat der Formel (II) mit mindestens einer Verbindung der Formel (III) umgesetzt wird.

7. Verwendung des Adduktes der Formel (I) gemäss einem der Ansprüche 1 bis 5 als Haftvermittler für Isocyanatgruppen enthaltenden Polymerzusammensetzungen.

8. Einkomponentige Zusammensetzung umfassend mindestens ein Addukt der Formel (I) gemäss einem der Ansprüche 1 - 5 sowie mindestens ein Polymer **P**, welches Isocyanatgruppen und gegebenenfalls Silangruppen aufweist.

9. Einkomponentige Zusammensetzung gemäss Anspruch 8, **dadurch gekennzeichnet, dass** das Polymer **P** ein Isocyanatgruppen aufweisendes Polyurethanpolymer **P1** ist, welches erhältlich ist aus der Reaktion von mindestens einem Polyisocyanat und mindestens einem Polyol.

10. Einkomponentige Zusammensetzung gemäss Anspruch 8, **dadurch gekennzeichnet, dass** das Polymer **P** ein sowohl Isocyanat- als auch Silangruppen aufweisendes Polyurethanpolymer **P2** ist, welches erhältlich ist
aus der Reaktion von
mindestens einem Isocyanatgruppen aufweisenden Polyurethanpolymer **P1,**
seinerseits erhältlich aus der Reaktion von mindestens
einem Polyisocyanat und mindestens einem Polyol;
und
einem Organoalkoxysilan mit mindestens einer Isocyanat-reaktiven Gruppe,
wobei das Organoalkoxysilan in Bezug auf die Isocyanatgruppen unterstöchiometrisch eingesetzt wird.

11. Einkomponentige Zusammensetzung gemäss Anspruch 10, **dadurch gekennzeichnet, dass** das Organoalkoxysilan mit mindestens einer Isocyanat-reaktiven Gruppe ein sekundäres Aminosilan oder ein Mercaptosilan ist.

12. Einkomponentige Zusammensetzung gemäss Anspruch 10 oder 11, **dadurch gekennzeichnet, dass** das Verhältnis von Isocyanatgruppen zu Silangruppen 10 bis 1, insbesondere 6 bis 2, beträgt.

13. Einkomponentige Zusammensetzung gemäss einem der Ansprüche 8 bis 12, **dadurch gekennzeichnet, dass** der Anteil des Adduktes der Formel (I) 0.1 bis 10 Gewichts-%, bevorzugt 0.3 bis 6 Gewichts-%, und insbesondere 0.5 bis 5 Gewichts-%, bezogen auf die einkomponentige Zusammensetzung, beträgt.

14. Einkomponentige Zusammensetzung gemäss einem der Ansprüche 9 bis 13, **dadurch gekennzeichnet, dass** das Polyol ein Polyoxyalkylenpolyol, insbesondere ein Polyoxypropylendiol oder -triol oder ein Ethylenoxidterminiertes Polyoxypropylendiol oder -triol, ist.

15. Einkomponentige Zusammensetzung gemäss Anspruch 14, **dadurch gekennzeichnet, dass** das Polyol ein Molekulargewicht von 1'000 bis 30'000 g/mol und einen Ungesättigtheitsgrad von tiefer als 0.02 mEq/g aufweist.

16. Einkomponentige Zusammensetzung gemäss einem der Ansprüche 9 bis 15, **dadurch gekennzeichnet, dass** das Polyisocyanat ausgewählt ist aus der Gruppe umfassend 1,6-Hexamethylendiisocyanat (HDI), 2,4- und 2,6-Toluylendiisocyanat (TDI), 4,4'-, 2,4'- und 2,2'-Diphenylmethandiisocyanat (MDI) und Isophorondiisocyanat (IPDI).

17. Zweikomponentige Zusammensetzung bestehend aus zwei Komponenten **K1** und **K2,**
wobei
die Komponente **K1** mindestens ein Addukt der Formel (I) gemäss einem der Ansprüche 1 - 5 sowie mindestens ein Polyisocyanat umfasst,
und
die Komponente **K2** mindestens ein Polyol und/oder mindestens ein Polyamin umfasst.

18. Zweikomponentige Zusammensetzung gemäss Anspruch 17, **dadurch gekennzeichnet, dass** das Polyisocyanat 4,4'-, 2,4'- oder 2,2'-Diphenylmethandiisocyanat (MDI) oder polymeres MDI (PMDI) oder eine bei Raumtemperatur flüssige Form von MDI oder ein Polyurethanpolymer **P1**, welches unter Verwendung von 1,6-Hexamethylendiisocyanat (HDI); oder 4,4'-, 2,4'- oder 2,2'-Diphenylmethandiisocyanat (MDI); oder 2,4- oder 2,6-Toluylendiisocyanat (TDI); oder 4,4'-, 2,4'- oder 2,2'-Diphenylmethandiisocyanat (MDI); oder Isophorondiisocyanat (IPDI) hergestellt wurde, ist.

19. Zweikomponentige Zusammensetzung gemäss Anspruch 17 oder 18, **dadurch gekennzeichnet, dass** der Anteil des Adduktes der Formel (I) 0.1 bis 10 Gewichts-%, bevorzugt 0.3 bis 6 Gewichts-%, und insbesondere 0.5 bis 5 Gewichts-%, bezogen auf die zweikomponentige Zusammensetzung, beträgt.

20. Gemischte zweikomponentige Zusammensetzung erhalten durch Mischen der Komponenten **K1** und **K2** einer zweikomponentigen Zusammensetzung gemäss einem der Ansprüche 17 bis 19, derart, dass die Isocyanatgruppen in Bezug auf die Hydroxyl- und/oder Aminogruppen im Überschuss eingesetzt werden, insbesondere derart, dass das Verhältnis ([OH] + [NH])/[NCO] einen Wert von 0.5 bis 0.95 aufweist.

21. Verfahren zur Herstellung einer einkomponentigen Zusammensetzung gemäss einem der Ansprüche 8 bis 16, **dadurch gekennzeichnet, dass** die Herstellung des Adduktes der Formel (I) und die Herstellung des Polymers **P** getrennt voneinander durchgeführt werden.

22. Verfahren zur Herstellung einer zweikomponentigen Zusammensetzung gemäss einem der Ansprüche 17 bis 19, **dadurch gekennzeichnet, dass** das Polyisocyanat nach der Herstellung des Adduktes der Formel (I) mit diesem vermischt wird.

23. Verfahren zur Verklebung umfassend die Schritte
a) Applikation einer einkomponentigen Zusammensetzung gemäss einem der Ansprüche 8 bis 16 oder einer gemischten zweikomponentigen Zusammensetzung gemäss Anspruch 20 auf ein Substrat **S1** und/oder ein Substrat **S2,**
b) Fügen der Fügeteile,
c) Aushärten der Zusammensetzung,
wobei das Substrat **S1** gleich oder verschieden von Substrat **S2** ist.

24. Verfahren zum Abdichten umfassend die Schritte
a) Applikation einer einkomponentigen Zusammensetzung gemäss einem der Ansprüche 8 bis 16 oder einer gemischten zweikomponentigen Zusammensetzung gemäss Anspruch 20 zwischen die Substrate **S1** und **S2,**
b) Aushärten der Zusammensetzung,
wobei das Substrat **S1** gleich oder verschieden von Substrat **S2** ist.

25. Verfahren gemäss Anspruch 23 oder 24, **dadurch gekennzeichnet, dass** mindestens eines der Substrate **S1** oder **S2** ein Lack, insbesondere ein Automobildecklack, oder eine lackierte Oberfläche ist.

26. Verfahren gemäss Anspruch 25, **dadurch gekennzeichnet, dass** der Lack nicht mit einem Primer vorbehandelt ist.

27. Verklebter oder abgedichteter Artikel erhalten durch ein Verfahren gemäss einem der Ansprüche 23 bis 26.

28. Verklebter oder abgedichteter Artikel gemäss Anspruch 27, **dadurch gekennzeichnet, dass** der Artikel ein Transportmittel, insbesondere ein Automobil, oder ein Anbauteil eines Transportmittels, insbesondere eines Automobils, ist.
